(19) Europäisches Patentamt
European Patent Office
Office européen des brevets

(11) EP 2 581 079 B1

(12) **EUROPEAN PATENT SPECIFICATION**

(45) Date of publication and mention of the grant of the patent:
**07.12.2016 Bulletin 2016/49**

(51) Int Cl.:
***A61K 9/06*** *(2006.01)* ***A61K 31/167*** *(2006.01)*
***A61K 47/36*** *(2006.01)*

(21) Application number: **11008211.2**

(22) Date of filing: **11.10.2011**

(54) **Combination of hyaluronic acid and prilocaine**

Kombination aus Hyaluronsäure und Prilocain

Combinaison d'acide hyaluronique et de prilocaïne

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**17.04.2013 Bulletin 2013/16**

(73) Proprietor: **BioPolymer GmbH & Co. KG**
**19073 Dümmer (DE)**

(72) Inventor: **Laeschke, Klaus**
**56235 Ransbach-Baumbach (DE)**

(74) Representative: **Brosch, Oliver et al**
**Kutzenberger Wolff & Partner**
**Theodor-Heuss-Ring 23**
**50668 Köln (DE)**

(56) References cited:
**EP-A1- 1 837 347**
**EP-A2- 0 466 300**
**WO-A1-2010/015901**
**US-A- 5 827 937**
**US-A1- 2006 122 147**
**US-A1- 2010 255 068**

- **JOHANSSON A ET AL: "EFFECT OF ADJUVANTS TO LOCAL ANAESTHETICS ON THEIR DURATION. IV. EFFECT OF HYALURONIC ACID ADDED TO BUPIVACAINE OR PRILOCAINE ONTHE DURATION OF NERVE BLOCKADE IN MAN", ACTA ANAESTHESIOLOGICA SCANDINAVICA, WILEY-BLACKWELL MUNKSGAARD, DK, vol. 29, no. 7, 1 January 1985 (1985-01-01), pages 736-738, XP000647890, ISSN: 0001-5172, DOI: 10.1111/J.1399-6576.1985.TB02291.X**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

The invention relates to a composition of matter comprising hyaluronic acid and/or a pharmaceutically acceptable hyaluronate salt, cross-linked hyaluronic acid and/or a pharmaceutically acceptable cross-linked hyaluronate salt, and prilocaine and/or a pharmaceutically acceptable salt thereof, and optionally at least one additive.

Hyaluronic acid (hyaluronan) is a naturally occurring long-chained, linear polysaccharide of the general formula $(C_{14}H_{21}NO_{11})_n$ representing repeating disaccharide units. Hyaluronic acid is usually present in form of its sodium salt and may have different chain lengths. In vivo, the molecular weight of the polymer strains is typically in the range of from 5,000 to 20,000,000 Da.

Hyaluronic acid can be isolated from various sources, for example, from human umbilical cord, cock's comb, or the connective tissue of vertebrates. Hyaluronic acid is also present in bacteria such as streptococci and may therefore also be obtained via fermentation processes.

Hyaluronic acid is biologically compatible and useful in numerous medical applications, e.g. in the field of ophthalmology, orthopedics, rheumatology, dermatology and cosmetic surgery. Hyaluronic acid is also used in cosmetic products, especially skin care and anti-aging products.

WO 2010/015901 A1 discloses cohesive soft tissue fillers, for example, dermal and subdermal fillers, based on hyaluronic acids and pharmaceutically acceptable salts thereof.

US 2010/255068 A1 provides methods for preparing crosslinked hydrogels for soft tissue augmentation.

It has been stated that hyaluronic acid is also useful in ophthalmology, when applied in combination with an anesthetic.

US 5,972,326 discloses a composition for intracameral use in ophthalmologic surgery comprising a viscoelastic polymer and an effective amount of an anesthetic agent. Hyaluronic acid is mentioned as an exemplary viscoelastic polymer.

WO 00/37047 discloses a composition for use in intraocular operations comprising a first and a second viscoelastic agent, characterized in that the first viscoelastic agent contains a concentration of at least one first anesthetic agent and in that the second viscoelastic agent contains a concentration of at least one second anesthetic.

US 6,224,857 discloses preparations consisting of a stoichiometrically neutral salt of a hyaluronic acid with a basic anesthetic, which are useful in medical applications such as corneal anesthesia.

A specific medical application of hyaluronic acid in the field of orthopedics and rheumatology is the intra-articular application. Its efficacy in the relief of pain and improvement in joint function has been shown in numerous clinical studies. It has been attributed to the interference of hyaluronic acid with the synovial cells, but also to a mechanical effect of lubrication of the cartilage structures.

Current methods of reducing pain in osteoarthritic joints include topical application of hyaluronic acid to the joint, and intra-articular injection of hyaluronic acid directly into the joint, and the treatment with analgesics or anti-inflammatory medications. The intra-articular application of a combination preparation of hyaluronic acid with another active ingredient has also been reported.

US 2006/0122147 discloses a combination preparation comprising an active substance A selected from the group consisting of hyaluronic acid and the salts and fragments thereof, at least one active substance B selected from the group consisting of local anesthetics and derivatives thereof, and other optional additives, and the use of the combination preparation in the treatment of degenerative and traumatic diseases of all joints, for the treatment of articular cartilage bone defects, and meniscus and invertebral disk lesions. As the only exemplary local anesthetic lidocaine hydrochloride is mentioned.

The most frequent adverse reactions to intra-articular injections in general are the experience of strong localized pain, the formation of articular effusions, and acute inflammation of the articular capsule due to injection-induced foreign body reaction. The same applies for intra-articular injections of hyaluronic acid and combination preparations thereof.

It is an object of the present invention to provide a composition comprising hyaluronic acid and/or a pharmaceutically acceptable salt thereof having advantages over the prior art.

The object is achieved by the inventive composition of matter described herein.

Thus, in one of its aspects, the present invention is directed to a composition of matter comprising,

(a1) hyaluronic acid and/or a pharmaceutically acceptable hyaluronate salt thereof, and
(a2) cross-linked hyaluronic acid and/or a pharmaceutically acceptable cross-linked hyaluronate salt, having a cross-linking degree of 35 to 65%, and
(b) prilocaine and/or a pharmaceutically acceptable salt thereof, and optionally
(c) at least one additive.

It has surprisingly been found that the combination of hyaluronic acid and/or a pharmaceutically acceptable hyaluronate salt, cross-linked hyaluronic acid and/or a pharmaceutically acceptable cross-linked hyaluronate salt and

prilocaine and/or a pharmaceutically acceptable salt thereof is useful in various medical indications, such as in the field of orthopedics and rheumatology.

It has surprisingly been found that, if the inventive combination is administered by injection, preferably intra-articular injection, the pain related to the injection or intra-articular injection can be reduced. It is particularly advantageous that prilocaine is somewhat less toxic in higher doses than lidocaine and that it is released quickly from the inventive composition of matter, ensuring a rapid onset of its beneficial action.

It has also surprisingly been found that, if the inventive combination is administered by injection, preferably intra-articular injection, acute inflammation and injection-induced foreign body reactions can be reduced. The effect can be attributed to the specific pharmacological properties of the inventive combination. In particular, the cross-linked hyaluronic acid and/or a pharmaceutically acceptable cross-linked hyaluronate salt seem to have a beneficial influence with regard to the pharmacodynamic properties of prilocaine.

It has also been found that the inventive combination is highly stable after injection, preferably intra-articular injection, allowing for an administration in prolonged intervals. This effect can be attributed to the cross-linked hyaluronic acid and/or a pharmaceutically acceptable cross-linked hyaluronate salt component in the inventive combination. In particular, a higher cross-linking degree allows for longer intervals between each injection.

The composition of matter according to the invention therefore surprisingly represents a new improved composition, which is particularly useful in the treatment of degenerative and traumatic diseases of all joints, for the treatment of articular cartilage bone defects, and meniscus and invertebral disk lesions, preferably when administered by intra-articular injection. The composition of matter is also useful in the treatment of arthrosis and articular rheumatism, and in the treatment of osteochondritis dissecans and flake fractures.

The composition of matter surprisingly also represents a new improved composition, which is particularly useful in the treatment of skin and mucous membrane changes, in cosmetic surgery, and in ophthalmologic surgery.

For the purpose of the specification, "hyaluronic acid" is intended to include polysaccharides of the general formula $(C_{14}H_{21}NO_{11})_n$, wherein n is typically in the range of 5,000 to 500,000, preferably 10,000 to 200,000, more preferably 20,000 to 100,000. The molecular formula $C_{14}H_{21}NO_{11}$ in the general formula $(C_{14}H_{21}NO_{11})_n$ represents a disaccharide unit composed of D-glucuronic acid and D-N-acetylglucosamide, linked via alternating β-1,4- and β-1,3-glycosidic bonds. Hyaluronic acid is also known as hyaluronan, or simply HA. It is known in the art that hyaluronic acid may be used as a pharmaceutically acceptable ingredient in pharmaceutical and cosmetic compositions.

For the purpose of the specification, "hyaluronic acid" unless otherwise stated is non-cross-linked hyaluronic acid, preferably unbranched hyaluronic acid of the general formula $(C_{14}H_{21}NO_{11})_n$.

Contrary thereto, "cross-linked hyaluronic acid" is obtainable by cross-linking hyaluronic acid of the general formula $(C_{14}H_{21}NO_{11})_n$ with a suitable cross-linking agent such as a diglycidyl ether. Suitable cross-linking agents are well known to those skilled in the art.

The pharmaceutically acceptable salts of hyaluronic acid are preferably salts, wherein the hyaluronic acid is present in at least partially deprotonated, i.e. anionic, form. Each disaccharide unit of hyaluronic acid contains a carboxylic acid function, which can be deprotonated. Preferably, at least the carboxylic acid function of one of the disaccharide units of hyaluronic acid is present in deprotonated form. Such a deprotonated disaccharide unit can be represented by the anionic molecular formula $(C_{14}H_{20}NO_{11})^-$.

Pharmaceutically acceptable salts of hyaluronic acid, wherein the hyaluronic acid is at least partially present in anionic form, are also referred to as hyaluronate salts, or simply hyaluronates. As used herein, a pharmaceutically acceptable hyaluronate salt of hyaluronic acid is defined as a salt of hyaluronic acid, wherein the carboxylic acid functions of the hyaluronic acid have been at least partially deprotonated, e.g. by means of a base such as sodium hydroxide, calcium hydroxide and/or potassium hydroxide. Suitable pharmaceutically acceptable hyaluronate salts include alkaline and alkaline earth metal salts, preferably sodium, potassium or calcium salts, more preferably sodium salts. Sodium hyaluronate can for example be represented by the general formula $(C_{14}H_{20}NaNO_{11})_n$, if the carboxylic acid function of each disaccharide unit has been at least partially deprotonated.

The terms "hyaluronic acid" and "a pharmaceutically acceptable salt of hyaluronic acid" as well as "hyaluronate" as used herein also cover pharmaceutically acceptable solvates, particularly hydrates.

The pharmaceutically acceptable salts of cross-linked hyaluronic acid are preferably salts, wherein the cross-linked hyaluronic acid is present in at least partially deprotonated, i.e. anionic, form. Each disaccharide unit of the cross-linked hyaluronic acid contains a carboxylic acid function, which can be deprotonated. Preferably, at least the carboxylic acid function of one of the disaccharide units of the cross-linked hyaluronic acid is present in deprotonated form.

Pharmaceutically acceptable salts of cross-linked hyaluronic acid, wherein the cross-linked hyaluronic acid is at least partially present in anionic form, are also referred to as cross-linked hyaluronate salts, or simply cross-linked hyaluronates. As used herein, a pharmaceutically acceptable cross-linked hyaluronate salt is defined as a salt of cross-linked hyaluronic acid, wherein the carboxylic acid functions of the cross-linked hyaluronic acid have been at least partially deprotonated, e.g. by means of a base such as sodium hydroxide, calcium hydroxide and/or potassium hydroxide. Suitable pharmaceutically acceptable cross-linked hyaluronate salts include alkaline and alkaline earth metal salts,

preferably sodium, potassium or calcium salts, more preferably sodium salts.

The terms "cross-linked hyaluronic acid" and "a pharmaceutically acceptable salt of cross-linked hyaluronic acid" as well as "cross-linked hyaluronate" as used herein also cover pharmaceutically acceptable solvates, particularly hydrates.

Prilocaine, also known as N-(2-methylphenyl)-2-(propylamino)propanamide, having the formula $C_{13}H_{20}N_2O$ contains a stereogenic center, and therefore exists in two different isomeric forms represented by formula I and I*:

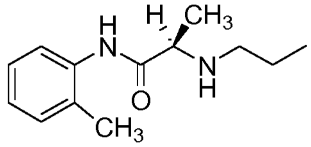

**I**

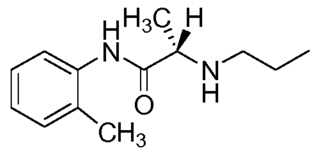

**I*.**

For the purpose of the specification, "prilocaine" is intended to include prilocaine in the form of the enantiomers, the racemate, or a mixture of the enantiomers in any desired mixing ratio. Preferably, prilocaine or the pharmaceutically acceptable salts thereof are used in racemic form (CAS number 721-50-6).

The pharmaceutically acceptable salts of prilocaine are preferably salts, wherein prilocaine is present in protonated, i.e. cationic, form. Suitable pharmaceutically acceptable salts include salts of inorganic acids, such as hydrochloric acid, hydrobromic acid, and sulfuric acid, and salts of organic acid, such as methane sulfonic acid, fumaric acid, maleic acid, acetic acid, oxalic acid, succinic acid, malic acid, tartaric acid, mandelic acid, lactic acid, citric acid, glutamic acid, acetylsalicylic acid, nicotinic acid, aminobenoic acid, $\alpha$-liponic acid, hippuric acid, and asparaginic acid. The preferred salt is the hydrochloride salt. More particularly racemic prilocaine hydrochloride (CAS number 1786-81-8) may be used in the inventive composition. The terms "prilocaine" and "a pharmaceutically acceptable salt of prilocaine" as used herein also cover pharmaceutically acceptable solvates, particularly hydrates.

For the purpose of the invention, doses of hyaluronic acid or prilocaine relate to the free acids or bases, respectively. Thus, when a pharmaceutically acceptable salt is used instead, its dose has to be adapted to the equivalent dose of the free acid or base. If not expressly stated otherwise, doses are "per administration".

For the purpose of the specification, the term "additive" is an additional ingredient, which influences the chemical, physical and/or pharmacological properties of the composition, such as the pH, the osmolality, the viscosity, and the like. Exemplary additives include buffering agents, osmolality adjusters, pH adjusters, thickening agents, gelling agents, preservatives, stabilizers, solubilizers, emulsifying agents, anti-oxidants, electrolytes, radical scavengers, vitamins, enzymes, scents, coloring agents, pigments, melanin, light protection filters, waxes, resins, oils, esters, alcohols, and polyols.

In a first aspect, the invention is directed to a composition of matter comprising,

(a1) hyaluronic acid and/or a pharmaceutically acceptable hyaluronate salt thereof, and
(a2) cross-linked hyaluronic acid and/or a pharmaceutically acceptable cross-linked hyaluronate salt, having a cross-linking degree of 35 to 65%, and
(b) prilocaine and/or a pharmaceutically acceptable salt thereof, and optionally
(c) at least one additive.

In one embodiment, the composition of matter according to the invention is an aqueous composition. Preferably, the composition comprises water in an amount of at least 90% by weight, preferably at least 95% by weight, more preferably at least 96% by weight based on the total weight of the composition.

In one embodiment, the composition of matter according to the invention has a dynamic viscosity (20°C, 10 revolutions per second) of 5,000 to 20,000 mPa*s, preferably 6,000 to 18,000 mPa*s, more preferably 8,000 to 16,000 mPa*s, and most preferably 8,000 to 12,000 mPa*s. The dynamic viscosity of the composition may for example be adjusted by varying the amount of water contained in the composition or by adding a thickener. The dynamic viscosity may be determined using standard methods known to those skilled in the art such as by using a rotation viscosimeter. The dynamic viscosity may preferably be measured with a rotational viscosimeter of the type Rheometer RS 1 with 10 revolutions per second at a temperature of 20°C. More preferably, a HAAKE RheoStress 1 rheometer available from Thermo Electron is used, wherein the dynamic viscosity is determined according to DIN 53019, 54453 and/or 3219.

In one embodiment, the composition has a pH value of 6.5 to 7.5, for example 6.9 to 7.5, or 7.0 to 7.4. The pH value may be determined using standard methods known in the art such as potentiometric methods. Preferably, the pH value may be determined by a potentiometric method according to NF T90-008 using a glass electrode.

In one embodiment, the composition has an osmolality of 280 to 360 mOsmol/l, for example 280 to 330 mOsmol/l

or 300 to 320 mOsmol/l. The osmolality may be determined using standard methods known to those skilled in the art such as measuring of the freezing point depression using a freezing point depression osmometer. Preferably, a K-7400 osmometer available from Knauer GmbH may be used for determining the osmolality of the composition.

Certain embodiments of the inventive composition of matter may be characterized by the following features according to E1 to E4:

| | E1 | E2 | E3 | E4 |
|---|---|---|---|---|
| amount of water based on the total weight of the composition | at least 90% by weight | at least 95% by weight | at least 97% by weight | at least 97 % by weight |
| dynamic viscosity | 5,000 to 20,000 mPa*s | 6,000 to 16,000 mPa*s | 8,000 to 16,000 mPa*s | 8,000 to 12,000 mPa*s |
| pH | 6.5-7.5 | 6.5-7.5 | 7.0-7.2 | 7.0-7.2 |
| osmolality | 280 to 360 mosmol/l | 280 to 330 mosmol/l | 300 to 320 mosmol/l | 300 to 320 mosmol/l |

In one embodiment, the composition of matter comprises component (a1) in an amount of 0.001 to 5% by weight, preferably 0.01 to 2% by weight, more preferably 0.01 to 0.5 % by weight based on the total weight of the composition.

In one embodiment, component (a1) has an average molecular weight of 2,200,000 to 3,800,000 Dalton, preferably 2,400,000 to 3,600,000, more preferably 2,500,000 to 3,500,000 Dalton. The average molecular weight may be determined using standard methods known in the art, such as those described by Ueno et al., 1988, Chem. Pharm. Bull. 36, 4971-4975; Wyatt, 1993, Anal. Chim. Acta 272, 1-40; and Wyatt Technologies, 1999, "Light Scattering University DAWN Course Manual" and "DAWN EOS Manual" Wyatt Technology Corporation, Santa Barbara. Calif.

In one embodiment, component (a1) is hyaluronic acid and/or a pharmaceutically acceptable hyaluronate salt thereof. Preferably, the hyaluronate salt according to component (a1) is an alkaline or alkaline earth metal hyaluronate. More preferably, the hyaluronate salt according to component (a1) is selected from the group consisting of sodium hyaluronate, potassium hyaluronate, calcium hyaluronate and any mixture thereof. Most preferably, the hyaluronate salt according to component (a1) is sodium hyaluronate.

In a preferred embodiment, component (a1) is sodium hyaluronate with an average molecular weight of 2,500,000 to 3,500,000 Dalton, more preferably 2,900,000 to 3,500,000. Component (a1) may be of any origin, such as animal origin, human origin or microbial origin.

In one embodiment, component (a1) is of non-animal and non-human origin. For example, component (a1) may be obtained by microbial fermentation. Preferably, the quality of component (a1) obtained by fermentation may be characterized by the following parameters: total viable aerobics count < 10 CFU/g and/or bacterial endotoxins < 0.025 I.U./mg and/or proteins < 0.03 % by weight.

In one embodiment, component (a1) is preferably recombinantly produced, more preferably by a Gram-positive bacterium or host cell, still more preferably by a bacterium of the genus Bacillus. In another embodiment, component (a1) is obtained from a Streptococcus cell.

The host cell may be any Bacillus cell suitable for recombinant production of hyaluronic acid. The Bacillus host cell may be a wild-type Bacillus cell or a mutant thereof. Bacillus cells useful in the practice of the present invention include, but are not limited to, Bacillus agaraderhens, Bacillus alkalophilus, Bacillus amyloliquefaciens, Bacillus brevis, Bacillus circulans, Bacillus clausii, Bacillus coagulans, Bacillus firmus, Bacillus lautus, Bacillus lentus, Bacillus licheniformis, Bacillus megaterium, Bacillus pumilus, Bacillus stearothermophilus, Bacillus subtilis, and Bacillus thuringiensis cells. Mutant Bacillus subtilis cells particularly adapted for recombinant expression are described in WO 98/22598.

The fermentation of the Bacillus subtilis A164Δ5 host to produce component (a1) is followed by a recovery process, during which only water-based solvents are employed. The final recovery step consists of spray-drying, which affords the final powder of component (a1).

Certain preferred embodiments of component (a1), which is preferably hyaluronic acid or a hyaluronate selected from sodium hyaluronate, potassium hyaluronate, and calcium hyluronate, may be characterized by the following features E5 to E8:

| | E5 | E6 | E7 | E8 |
|---|---|---|---|---|
| Average Molecular weight [Dalton] | 2,200,000 to 3,800,000 | 2,400,000 to 3,600,000 | 2,500,000 to 3,500,000 | 2,500,000 to 3,500,000 |

(continued)

| | E5 | E6 | E7 | E8 |
|---|---|---|---|---|
| Origin | Human, animal, or microbial origin | Fermentation of a bacterial strain | Fermentation of a Bacillus cell | Fermentation of a Bacilus subtilis A164Δ5 cell |

In one embodiment, the composition of matter comprises component (a2) in an amount of 0.01 to 10% by weight, preferably 0.1 to 3% by weight, more preferably 0.5 to 2.5% by weight. Preferably, component (a2) is contained in a higher amount than component (a1).

In one embodiment, component (a2) has an average molecular weight of 500,000 to 2,000,000, preferably 600,000 to 1,800,000, more preferably 700,000 to 1,500,000 Dalton, most preferably 900,000 to 1,100,000 Dalton. The average molecular weight of may be determined using standard methods in the art, such as those described by Ueno et al., 1988, Chem. Pharm. Bull. 36, 4971-4975; Wyatt, 1993, Anal. Chim. Acta 272, 1-40; and Wyatt Technologies, 1999, "Light Scattering University DAWN Course Manual" and "DAWN EOS Manual" Wyatt Technology Corporation, Santa Barbara. Calif.

In one embodiment, component (a2) is preferably cross-linked hyaluronic acid, cross-linked sodium hyaluronate, cross-linked potassium hyaluronate, and/or cross-linked calcium hyaluronate. Preferably, component (a2) is cross-linked sodium hyaluronate.

In one embodiment, component (a2) is cross-linked hyaluronic acid, cross-linked hyaluronate and/or a mixture thereof, which preferably has been cross-linked with a diglycidyl ether, more preferably with 1,4-butanediol diglycidyl ether (BDDE), as cross-linking agent.

In one embodiment, component (a2) is cross-linked sodium hyaluronate, cross-linked potassium hyaluronate, and/or cross-linked calcium hyaluronate, which has been cross-linked with a diglycidyl ether, preferably with 1,4-butanediol diglycidyl ether (BDDE), as cross-linking agent.

In one embodiment, component (a2) is the reaction product of sodium hyaloronate and BDDE. Preferably, the hydroxyl groups of the disaccharide units of sodium hyaluronate are cross-linked via 1,1'-[butane-1,4-diylbis(oxy)]dipropan-2-ol-bridges, i.e. $-CH_2-CHOH-CH_2-O-CH_2-CH_2-CH_2-CH_2-O-CH_2-CHOH-CH_2-$bridges. More preferably, component (a2) is represented by the formula $(C_{14}H_{20-2m}NNaO_{11})_n(C_{10}H_{20}O_4)_m(H_2O)$, wherein n is preferably in the range of 10,000 to 200,000, preferably 20,000 to 100,000, and m is in the range of 5,000 to 100,000, preferably 10,000 to 50,000.

In one embodiment, component (a2) has a cross-linking degree of preferably 40 to 60%, more preferably 42 to 55%. The more cross-linking agent is involved in the cross-linking of the hyaluronic acid and/or hyaluronate, i.e. the more cross-linking agent bridges are formed, the higher is the cross-linking degree. Typically, only the primary hydroxyl groups of the disaccharide units in hyaluronic acid and/or hyaluronate are cross-linked. Since the disaccharide unit comprises only one primary hydroxyl group, the maximum number of possible cross-linking agent bridges is limited to 1 bridge per disaccharide unit. If all primary hydroxyl groups of the hyaluronic acid and/or hyaluronate are linked to a cross-linking agent bridge, the cross-linking degree is 100%. In general, the cross-linking degree (CD) is defined by formula 1:

$$CD\ (\%) = \frac{N_{bridges} \times 100}{N_{max}}\ \% \qquad (1),$$

wherein $N_{bridges}$ = Total number of introduced cross-linking agent bridges
$N_{max}$ = Maximum number of possible cross-linking agent bridges

The cross-linking degree may be determined by experimental methods involving elemental analysis of a sample of component (a2). Since the cross-linking agent bridge typically does not comprise nitrogen, the nitrogen/carbon ratio (N/C-ratio) gives information about the cross-linking degree. The lower the N/C-ratio is the higher is the cross-linking degree. The exact cross-linking degree may be determined with reference samples by means of calibration.

The cross-linking degree (CD) of a sample of component (a2), which is obtainable by cross-linking sodium hyaluronate with BDDE as cross-linking agent, may be determined with the following formula 2:

$$CD(\%) = \frac{\dfrac{A_c - (A_N/M_N) \times C_{Hyal} \times M_c}{C_{BDDE} \times M_c}}{\dfrac{(A_N/M_N) - Äq_{Hyal}}{2}} \times 100 \qquad (2),$$

wherein AN = Amount of nitrogen in the sample [%]
Ac = Amount of carbon in the sample [%]
$C_{Hyal}$ = Number of carbon atoms in a disaccharide unit involved in bridging
$C_{BDDE}$ = Number of carbon atoms in a BDDE-cross-linking bridge
$M_N$ = Atomic mass of nitrogen = 14.0067
$M_C$ = Atomic mass of carbon = 12.0107
$Äq_{Hyal}$ = molar amount of non-cross-linked hyaluronic acid [mol]

wherein the amounts of nitrogen and carbon (AN and $A_C$) in the sample may be determined by elemental analysis, preferably using a CHNSO-analyzer, such as Euro EA CNS available from HEKAtech GmbH;
wherein, the number of carbon atoms in a BDDE-cross-linking bridge $C_{BDDE}$ is represented by the number of carbon atoms in the molecular formula of the BDDE-bridge, $-(C_{10}H_{20}O_4)-$, i.e. $C_{BDDE}$ = 10;
wherein the number of carbon atoms in the disaccharide unit involved in bridging, is calculated by dividing the number of carbon atoms in the disaccharide unit represented by the molecular formula $-(C_{14}H_{20\text{-}2m}NNaO_{11})-$, i.e. 14, by 2 because two disaccharide units, each comprising only one reactive hydroxyl group, are reacted with one BDDE molecule, comprising two reactive groups; thus, $C_{Hyal}$ = 7; and
wherein $Äq_{Hyal}$ is typically 0 or equals the molar amount of hyaluronic acid in the sample, which optionally has been added to the sample after cross-linking.

If the cross-linking degree is determined by any of the above described methods, wherein elemental analysis is involved, the CHNSO-analyzer is preferably suitable for performing CHNS-analysis in the presence of water. The sample may comprise water in an amount of up to 90%, and the water is removed by heating before the sample is burnt and the CHNSO-content of the sample is determined in the gaseous phase by means of gas chromatography.

In a preferred embodiment, component (a2) is BDDE-cross-linked sodium hyaluronate with an average molecular weight of 700,000 to 1,500,000 Dalton, preferably 900,000 to 1,100,000 Dalton, and a cross-linking degree of 42-55%.

The hyaluronic acid and/or hyaluronate salt, which is cross-linked to component (a2), may be of any origin, such as animal origin, human origin or microbial origin.

In one embodiment, the hyaluronic acid and/or hyaluronate salt is of non-animal and non-human origin. Instead, the hyaluronic acid and/or hyaluronate salt to be cross-linked to component (a2) may be obtained by microbial fermentation as described for component (a1). Preferably, the quality of the hyaluronic acid and/or hyaluronate salt to be cross-linked is characterized by the following parameters: total viable aerobics count < 10 CFU/g and/or bacterial endotoxins < 0.025 I.U./mg and/or proteins < 0.03 % by weight.

Component (a2) may have a mean particle size in the range of 50 to 150 μm, 250 to 550 μm, or 850 to 1300 μm. High particle sizes, i.e. 850 to 1300 μm, preferably 900 to 1250 μm are preferred, if the composition of matter comprising (a2) is supposed to be useful in cosmetic applications or cosmetic surgery, e.g. for volume replacements. Low particle sizes, i.e. 50 to 150 μm, preferably 80 to 120 μm are preferred, if the composition of matter comprising (a2) is supposed to be useful in intra-articular applications, e.g. for the treatment of osteoarthritis.

In one embodiment, component (a2) has a mean particle size in the range of 50 to 150 μm, preferably 80 to 120 μm. Preferably, at least 90 vol.-%, preferably 99 vol.-% of the particles of component (a2), based on the total volume of component (a2) in the composition, have a diameter below 500 μm, preferably 300 μm, more preferably 250 μm, most preferably 200 μm.

In one embodiment, component (a2) has a particle size distribution characterized in that at least 80 vol.-% of the particles have a diameter in the range of 50 to 250 μm, at least 50 vol.-% of the particles have a diameter in the range of 50 to 150 μm, and at least 30 vol.-% of the particles have a diameter in the range of 80 to 120 μm. Preferably, component (a2) has a particle size distribution characterized in that at least 90 vol.-% of the particles have a diameter in the range of 50 to 250 μm, at least 70 vol.-% of the particles have a diameter in the range of 50 to 150 μm, and at least 50 vol.% of the particles have a diameter in the range of 80 to 120 μm, wherein the vol.-% is based on the total volume of component (a1) in the composition.

In certain embodiments, component (a2) is characterized by one of the following particle size distributions according to E9 or E10:

| | D (0.05)* | D (0.1)* | D (0.5) * | D (0.9) * | D(0.95)* |
|---|---|---|---|---|---|
| E9 | 45 μm ± 5% | 52 μm ± 5% | 120 μm ± 5% | 247 μm ± 5% | 300 μm ± 5% |
| E10 | 50 μm ± 5% | 60 μm ± 5% | 120 μm ± 5% | 150 Nm ± 3% | 250 μm ± 3% |
| *D (0.05), D (0.1), D (0.5), D (0.9), D (0.95) are the diameters, where 5 vol.-%, 10 vol.-%, 50 vol.-%, 90 vol.-% and 95 vol.-% of the particles have a smaller equivalent diameter than the indicated diameter. | | | | | |

The particle size distribution may be determined using standard methods used in the art, such as the laser diffraction technique. Laser diffraction, alternatively referred to as Low Angle Laser Light Scattering (LALLS), can be used for the non-destructive analysis of wet or dry samples, with particles in the size range of 0.02 to 2000 μm. Laser diffraction based particle size analysis relies on the fact that particles passing through a laser beam will scatter light at an angle that is directly related to their size. As particle size decreases, the observed scattering angle increases logarithmically. Scattering intensity is also dependent on particle size, diminishing with particle volume. Large particles therefore scatter light at narrow angles with high intensity whereas small particles scatter at wider angles but with low intensity. A typical system consists of a laser, to provide a source of coherent, intense light of fixed wavelength; a series of detectors to measure the light pattern produced over a wide range of angles; and some kind of sample presentation system to ensure that material under test passes through the laser beam as a homogeneous stream of particles in a known, reproducible state of dispersion. The dynamic range of the measurement is directly related to the angular range of the scattering measurement, with modern instruments making measurements from around 0.02 degrees through to beyond 140 degrees. The wavelength of light used for the measurements is also important, with smaller wavelengths (e.g. blue light sources) providing improved sensitivity to sub-micron particles. The Mastersizer 2000 available from Malvern Instruments GmbH, for example, is a fully optimized laser diffraction instrument which allows manufacturers to simply and rapidly generate high quality data on the particle size distribution of a compound. The particle size distribution in the composition may preferably be determined according to ISO 13320:2009 using a Mastersizer 2000 available from Malvern Instruments GmbH.

The desired particle size distribution can be achieved by filtering component (a2) via a sieve with variable pore size so that after filtration component (a2) exhibits a homogeneous particle size distribution. In addition, milling and/or crushing may be applied to reduce the particle size.

In one embodiment, component (a2) is obtainable by

(1) cross-linking hyaluronic acid and/or hyaluronate in solution to a cross-linking degree of 35 to 65%,
(2) filtering and washing the cross-linked hyaluronic acid and/or cross-linked hyaluronate obtained according to step (1),
(3) increasing the surface area of the cross-linked hyaluronic acid and/or cross-linked hyaluronate obtained according to step (2),
(4) further cross-linking the cross-linked hyaluronic acid and/or cross-linked hyaluronate obtained according to step (3) in the presence of hyaluronic acid and/or hyaluronate, and
(5) washing the cross-linked hyaluronic acid and/or cross-linked hyaluronate obtained according to step (4).

Preferably, the hyaluronic acid, which is cross-linked in step (1), carries hydroxyl and carboxyl groups that enable cross-linking by linking hydroxyl groups via ether bonds and carboxyl groups via ester bonds. If necessary, the hyaluronic acid can be chemically modified prior to cross-linking to introduce additional reactive functional groups. For example, hyaluronic acid can be partially deacetylated and thus amino groups can be cross-linked via amide, imine or amine bonds.

Preferably, the cross-linked hyaluronic acid and/or cross-linked hyaluronate obtained according to step (1) is cross-linked via ether- and/or ester- and/or sulfon- and/or amine- and/or imine- and/or amide-bonds, preferably via formation of ether- and/or ester- and/or sulfon-bonds, more preferably via ether- or sulfon-bonds in the presence of a cross-linking agent selected from the group consisting of formaldehyde, glutaraldehyde, divinyl sulfone, polyanhydrides, polyaldehydes, polyhydric alcohols, carbodiimides, carboxylic acid chlorides, sulfonic acid chlorides, epichlorohydrin, ethylene glycol, diglycidyl ethers such as butanediol diglycidyl ether, polyethylene glycol diglycidyl ether, and polypropylene gyclol diglycidyl ether, polyglycerol polyglycidyl ethers, and bis- or polyepoxides, preferably in the presence of butanediol diglycidyl ether or divinyl sulfone.

Preferably, the cross-linked hyaluronic acid and/or cross-linked hyaluronate obtained according to step (4) is cross-linked via ether- and/or ester- and/or sulfon- and/or amine- and/or imine- and/or amide-bonds, preferably via formation of ether- and/or ester- and/or sulfon-bonds, more preferably via ether- or sulfon-bonds in the presence of a

cross-linking agent selected from the group consisting of formaldehyde, glutaraldehyde, divinyl sulfone, polyanhydrides, polyaldehydes, polyhydric alcohols, carbodiimides, carboxylic acid chlorides, sulfonic acid chlorides, epichlorohydrin, ethylene glycol, diglycidyl ethers such as butanediol diglycidyl ether, polyethylene glycol diglycidyl ether, and polypropylene gyclol diglycidyl ether, polyglycerol polyglycidyl ethers, and bis- or polyepoxides, preferably in the presence of butanediol diglycidyl ether or divinyl sulfone.

Suitable bisepoxides and polyepoxides that can be used in the present invention are selected from the group consisting of ethylenglycole diglycidylether, (1,6)-hexandiol diglycidylether, polytetramethylenediglycole diglycidylether, neopentylglycole diglycidylether, polyglycerole polyglycidylether, diglycerole polyglycidylether, glycerole polyglycidylether, trimehtylolpropane polyglycidylether, pentaerythritol polyglycidylether, sorbitol polyglycidylether, (1,2,3,4)-diepoxybutane and (1,2,7,8)-diepoxyoctane.

Cross-linking of hyaluronic acid and/or hyaluronate via ether bonds is preferably achieved by using a cross-linking agent selected from the group consisting of formaldehyde, glutaraldehyde and divinylsulfone. Ether bonds can also be formed under alkaline conditions, preferably at a pH value > 10, more preferably at a pH value in the range between 10 and 12, by using a cross-linking agent selected from the group consisting of butanediol diglycidylether, diglycidyl ether, polyglycerole polyglycidylether, polyethyleneglycole diglycidylether, polypropyleneglycole diglycidylether, bisepoxides and polyepoxides, preferably selected from the group consisting of (1,2,3,4)-diepoxybutane and (1,2,7,8)-diepoxyoctane.

Cross-linking of hyaluronic acid and/or hyaluronate via ester bonds is preferably achieved by using a cross-linking agent selected from the group consisting of polyhydric alcohols, carbodiimides, polyanhydrides and carbonic acid chlorides. Ester bonds can also be formed under acidic conditions, preferably at a pH value < 4, more preferably at a pH value in the range between 2 and 4, by using a cross-linking agent selected from the group consisting of bisepoxides and polyepoxides preferably selected from the group consisting of (1,2,3,4)-diepoxybutane and (1,2,7,8)-diepoxyoctane.

Cross-linking of hyaluronic acid and/or hyaluronate via amide bonds is preferably achieved by using carbodiimides in the presence of at least one amine, at least one carbonic acid anhydride or at least one carbonic acid chloride in the case of deacetylated hyaluronic acid and at least one diisocyanate.

In the case of deacetylated hyaluronic acid and/or hyaluronate with amino groups, cross-linking of hyaluronic acid and/or hyaluronate via amine bonds is preferably achieved by using at least one epoxide or glutaraldehyde in the presence of a reducing agent.

In the case of deacetylated hyaluronic acid and/or hyaluronate, cross-linking of hyaluronic acid and/or hyaluronate via amine bonds is preferably achieved by using glutaraldehyde.

As used herein, the term "deacetylated hyaluronic acid and/or hyaluronate" relates to hyaluronic acid and/or hyaluronate, wherein the D-N-acetylglucosamide component in at least 1% of the disaccharide units has been deacetylated. Preferably, the acetylglucosamide component has been deacetylated in at least 10%, more preferably at least 50%, still more preferably at least 90%, most preferably at least 99% of the disaccharide units. Cross-linking of hyaluronic acid and/or hyaluronate via sulfone bonds is preferably achieved by using sulfonic acid chloride.

The weight ratio of cross-linking agent to hyaluronic acid and/or hyaluronate is preferably in the range between 1:10 and 10:1 based on the weight of the cross-linking agent and the weight of the hyaluronic acid and/or hyaluronate.

Preferably, filtering of the cross-linked hyaluronic acid and/or hyaluronate according to step (2) is performed via a sieve with variable pore size so that after filtration component (a2) exhibits a homogeneous particle size distribution. Preferably, washing of the cross-linked hyaluronic acid and/or hyaluronate according to step (2) is performed with water as the washing agent.

Preferably, increasing of the surface area of the cross-linked hyaluronic acid according to step (3) is achieved by milling and/or crushing.

Preferably, washing of the cross-linked hyaluronic acid and/or hyaluronate according to step (5) is performed with water as the washing agent.

Certain embodiments of component (a2), which is preferably a cross-linked hyaluronate selected from cross-linked sodium hyaluronate, cross-linked potassium hyaluronate, and cross-linked calcium hyluronate, most preferably cross-linked sodium hyaluronate, may be characterized by the following features E11 to E13:

| | E11 | E12 | E13 |
|---|---|---|---|
| average molecular weight [Dalton] | 500,000 to 2,000,000 | 600,000 to 1,800,000 | 700,000 to 1,500,000, preferably 900,000 to 1,100,000 |

(continued)

| | E11 | E12 | E13 |
|---|---|---|---|
| particle size distribution of particles having a diameter > 0.02 μm | Lower than 300 μm: > 95 vol.-%<br>Lower than 250 μm: > 90 Vol.-%<br>Lower than 120 μm: > 50 vol.-%<br>Lower than 50 μm: > 10 vol.-%<br>Lower than 45 μm: > 5 vol.-% | Lower than 250 μm: > 95 vol.-%<br>Lower than 150 μm: > 90 Vol.-%<br>Lower than 120 μm: > 50 vol.-%<br>Lower than 60 μm: > 10 vol.-%<br>Lower than 50 μm: > 5 vol.-% | Lower than 150 μm: > 95 vol.-%<br>Lower than 120 μm: > 90 Vol.-%<br>Lower than 100 μm: > 50 vol.-%<br>Lower than 90 μm: > 10 vol.-%<br>Lower than 70 μm: > 5 vol.-% |
| origin | animal, human or microbial origin | Fermentation of a bacillus cell | Fermentation of a bacilus subtilis A164Δ5 cell |
| cross-linking degree | 35-65% | 40-60% | 42-55% |
| cross-linking agent | divinyl sulfone or a diglycidyl ether | a diglycidyl ether | BDDE |

In one embodiment, the composition of matter comprises component (b) in an amount of 0.001 to 5% by weight, preferably 0.01 to 3% by weight, more preferably 0.1 to 1% by weight based on the total weight of the composition.

In one embodiment, component (b) is essentially dissolved in the composition of matter at room temperature. The dissolution of component (b) at room temperature may be determined by laser diffraction. The detection limit of laser diffraction is typically 0.02 μm. As used herein, "solution of the component (b)" is defined in that no particles of the respective component are detectable by means of laser diffraction, i.e. the solution does not contain more than 10 vol.-%, preferably not more than 5 vol.-%, more preferably not more than 1 vol.-%, most preferably not more than 0.1 vol.% particles of the respective component having a particle size, which is greater than 0.02 μm, wherein the vol.-% is based on the total volume of component (b) in the composition.

In one embodiment component (b) is a pharmaceutically acceptable salt of racemic prilocaine. Preferably, component (b) is racemic prilocaine hydrochloride.

In one embodiment, the composition of matter comprises component (c), which is one additive or a combination of additives, in an amount of 0.01 to 10% by weight, preferably 0.1 to 3% by weight and more preferably 0.5 to 1.5% by weight based on the total weight of the composition.

In one embodiment, the composition of matter comprises as component (c) one or more additives selected from the group consisting of buffering agents, osmolality adjusters, pH adjusters, thickening agents, gelling agents, preservatives, stabilizers, solubilizers, emulsifying agents, anti-oxidants, electrolytes, radical scavengers, vitamins, enzymes, scents, coloring agents, pigments, melanin, light protection filters, waxes, resins, oils, esters, alcohols, and polyols. Preferably, said additives may have a particle size distribution in the composition of matter characterized in that more than 90 vol.-%, preferably more than 95 vol.-%, still more preferably more than 99 vol.-%, most preferably 99.9 vol.-% of the particles have a diameter, which is lower than 10 μm, preferably 5 μm, more preferably 1 μm, still more preferably 0.1 μm, most preferably 0.02 μm, wherein the vol.-% is based on the total volume of component (c) in the composition.

A person skilled in the art understands that component (c) is typically provided as a dispersion, solution or mixture in water, when preparing the composition of matter. Preferably, component (c) is employed as an aqueous solution. Preferably, component (c) can be essentially dissolved in water, i.e. preferably no particles of component (c) are detectable by laser diffraction, as defined above. Thus, component (c) preferably represents an additive or a combination of additives, which is water soluble. "Water soluble" herein means that the additive can be essentially dissolved in water in an amount of at least 0.05% by weight based on the total weight of the solution, wherein the dissolution may be determined by laser diffraction, as defined above. Preferred water soluble additives, which may be present alone or in combination, are buffering agents, osmolality adjusters and pH adjusters.

In one embodiment, the composition of matter comprises as component (c) at least a buffering agent. Preferably, the composition of matter comprises as component (c) at least a buffering agent and an osmolality adjuster. More

preferably, the composition of matter comprises as component (c) at least a buffering agent, an osmolality adjuster, and a pH adjuster. Optionally, the composition of matter additionally comprises one or more additives selected from the group consisting of thickening agents, gelling agents, preservatives, stabilizers, solubilizers, emulsifying agents, anti-oxidants, electrolytes, radical scavengers, vitamins, enzymes, scents, coloring agents, pigments, melanin, light protection filters, waxes, resins, oils, esters, alcohols, and polyols.

Preferably, the buffering agent comprises an acid and/or the corresponding alkaline or alkaline earth metal salt thereof. Exemplary buffering agents include at least one acid or salt selected from the group consisting of sodium dihydrogen phosphate, disodium hydrogen phosphate ($Na_2HPO_4$), also referred to as disodium phosphate, trisodium phosphate, citric acid, sodium citrate, sodium hydrogen carbonate, sodium bicarbonate, tromethamine, potassium citrate, ascorbic acid, sodium ascorbate, potassium ascorbate, acetic acid, sodium acetate, lactobionic acid, sodium lactobionate, an amino acid, a salt of an amino acid, the hydrates of any of the listed acids and salts, and combinations thereof. Preferred buffering agents comprise disodium hydrogen phosphate and/or sodium dihydrogen phosphate and/or the hydrates thereof. Exemplary hydrates include monohydrate, dihydrate, heptahydrate, dodecahydrate. Most preferably the buffering agent is a mixture of disodium hydrogen phosphate dihydrate and sodium dihydrogen phosphate dihydrate.

Preferably, the osmolality adjuster comprises at least one alkaline or alkaline earth metal salt. Exemplary osmolality adjusters include at least one salt selected from sodium chloride, sodium acetate, sodium malate, sodium lactate, potassium chloride, potassium acetate, potassium malate, potassium lactate, calcium chloride, calcium acetate, calcium malate, calcium lactate, magnesium chloride, magnesium acetate, magnesium malate, and magnesium lactate, and combinations thereof. Preferred osmolality adjusters comprise sodium chloride. Most preferably, the osmolarity adjuster is sodium chloride.

Preferably, the pH adjuster comprises either an inorganic acid or a base. Preferably, the pH adjuster comprises either sodium hydroxide or hydrogen chloride. More preferably, the pH adjuster is an aqueous solution comprising either sodium hydroxide or hydrogen chloride. Most preferably, the pH adjuster is an aqueous solution comprising either $1\pm0.5\%$ by weight of sodium hydroxide or an aqueous solution comprising $1\pm0.5\%$ by weight of hydrogen chloride, wherein the wt.-% are based on the total weight of the aqueous solutions. It is particularly preferred that the pH adjuster is an aqueous solution comprising 1% by weight of sodium hydroxide or an aqueous solution comprising 1% by weight of hydrogen chloride, wherein the wt.-% are based on the total weight of the aqueous solutions.

In one embodiment, the composition of matter comprises as component (c) at least

- a buffering agent selected from the group consisting of disodium hydrogen phosphate dihydrate, sodium dihydrogen phosphate dihydrate and any mixture thereof.

Preferably, the composition of matter comprises as component (c) at least

- a buffering agent selected from the group consisting of disodium hydrogen phosphate dihydrate, sodium dihydrogen phosphate dihydrate and any mixture thereof, and
- sodium chloride as an osmolality adjuster.

More preferably, the composition of matter comprises as component (c) at least

- a buffering agent selected from the group consisting of disodium hydrogen phosphate dihydrate, sodium dihydrogen phosphate dihydrate and any mixture thereof, and
- sodium chloride as an osmolality adjuster,
- sodium hydroxide or hydrogen chloride as pH adjuster.

In one embodiment, the composition of matter comprises as component (c) at least

- a mixture of disodium phosphate dihydrate and sodium dihydrogen phosphate dihydrate, and
- sodium chloride.

Preferably, the composition of matter comprises as component (c) at least

- a mixture of disodium hydrogen phosphate dihydrate and sodium dihydrogen phosphate dihydrate,
- sodium chloride, and
- an aqueous solution comprising either $1\pm0.5\%$ by weight of sodium hydroxide or $1\pm0.5\%$ by weight of hydrogen chloride, wherein the wt.-% are based on the total weight of the aqueous solutions.

In one embodiment, the composition of matter comprises as component (c) at least

- a mixture of disodium hydrogen phosphate dihydrate and sodium dihydrogen phosphate dihydrate, and
- sodium chloride
  in a ratio of (2±1.5) : (10±2), preferably (1±0.5) : (10±1), more preferably (1±0,2) : (9±0.3) based on the weight of the additives.

In a preferred embodiment, component (c) represents one or more additives, selected from the group consisting of buffering agents, osmolality adjusters, pH adjusters, thickening agents, gelling agents, preservatives, stabilizers, solubilizers, emulsifying agents, anti-oxidants, electrolytes, radical scavengers, vitamins, enzymes, scents, coloring agents, pigments, melanin, light protection filters, waxes, resins, oils, esters, alcohols, and polyols, wherein at least one additive is a buffering agent, which is preferably a combination of disodium phosphate dihydrate and sodium dihydrogen phosphate dihydrate, and optionally at least one further additive is an osmolarity adjuster, which is preferably sodium chloride.

The invention also relates to a composition of matter comprising

(a1) hyaluronic acid and/or a pharmaceutically acceptable salt thereof in an amount of 0.01 to 0.5% by weight based on the total weight of the composition,
(a2) cross-linked hyaluronic acid and/or a cross-linked hyaluronate thereof, having a cross-linking degree of 35 to 65%, in an amount of 0.5 to 2.5% by weight based on the total weight of the composition,
(b) prilocaine or a pharmaceutically acceptable salt thereof in an amount of 0.1 to 1% by weight based on the total weight of the composition, and optionally
(c) one or more additives together accounting for an amount of 0.5 to 5% by weight based on the total weight of the composition,
and water in an amount of at least 90% by weight based on the total weight of the composition.

In another embodiment, the invention relates to a composition of matter comprising

(a1) sodium hyaluronate in an amount of 0.01 to 0.5% by weight based on the total weight of the composition,
(a2) cross-linked sodium hyaluronate, having a cross-linking degree of 35 to 65%, in an amount of 0.5 to 2.5% by weight based on the total weight of the composition,
(b) prilocaine hydrochloride in an amount of 0.1 to 1% by weight based on the total weight of the composition, and
(c) at least an osmolarity adjuster in an amount of 0.5 to 1.5% by weight based on the total weight of the composition, and a buffering agent in an amount of 0.05 to 0.15% by weight based on the total weight of the composition,
and water in an amount of at least 90% by weight based on the total amount of the composition.

In one embodiment, the invention relates to a composition of matter comprising
(a1) hyaluronic acid and/or a pharmaceutically acceptable hyaluronate salt,
(a2) cross-linked hyaluronic acid and/or a pharmaceutically acceptable cross-linked hyaluronate salt, having a cross-linking degree of 35 to 65%,
(b) prilocaine and/or a pharmaceutically acceptable salt thereof, and optionally
(c) at least one additive,
which is characterized in that it has a dissolution profile that releases component (b), i.e. prilocaine and/or a pharmaceutically acceptable salt thereof, according to the following in vitro dissolution profile indicated in table A:

Table A

| time [min] | in-vitro-dissolution of component (b) [wt.-%] |
|---|---|
| 5 | ≥ 10 |
| 10 | ≥ 20 |
| 20 | ≥ 30 |
| 30 | ≥ 40 |
| 45 | ≥ 50 |
| 60 | ≥ 60 |
| 90 | ≥ 70 |
| 120 | ≥ 80 |

(continued)

| time [min] | in-vitro-dissolution of component (b) [wt.-%] |
|---|---|
| 150 | ≥ 85 |
| 180 | ≥ 88 |
| 210 | ≥ 90 |
| 240 | ≥ 92 |

wherein the dissolution is preferably determined using tubes and dialysis as cavities, and wherein preferably one or more of the following may apply:

| | |
|---|---|
| Instrument: | Dissolution tester Sotax AT 7 smart or comparable |
| Sample: | 1 g in 9 cm dialyse tube Spectra/Por |
| Device: | Paddle |
| Rate: | 75 rpm |
| Medium: | Buffer solution pH = 6.8 R; containing $Na_2HPO_4$ x 12 $H_2O$ dissolved in water R (71.5 g/L) and Citric acid x 1 $H_2O$ dissolved in water R (21 g/L) and |
| Volume: | 500 mL |
| Temperature: | 37°C±0.5°C |
| Removal volume: | 2.5 mL |
| Profile [min]: | 10/45/120 for routine testing |

and wherein component (b) is preferably detected with UV detection at 230 nm according to the method of external standard.

In a preferred embodiment, the invention relates to a composition of matter comprising

(a1) hyaluronic acid and/or a pharmaceutically acceptable salt thereof in an amount of 0.01 to 0.5% by weight based on the total weight of the composition,
(a2) cross-linked hyaluronic acid and/or a cross-linked hyaluronate thereof, having a cross-linking degree of 35 to 65%, in an amount of 0.5 to 2.5% by weight based on the total weight of the composition,
(b) prilocaine or a pharmaceutically acceptable salt thereof in an amount of 0.1 to 1% by weight based on the total weight of the composition, and optionally
(c) one or more additives together accounting for an amount of 0.5 to 5% by weight based on the total weight of the composition,
which is characterized in that it has a dissolution profile that releases component (b), i.e. prilocaine and/or a pharmaceutically acceptable salt thereof, according to the in vitro dissolution profile as indicated in table A above.

The invention also relates to a composition of matter, which is obtainable by

(i) dissolving component (b) in an aqueous solution comprising as component (c1) at least one additive,
(ii) dispersing component (a1) in an aqueous solution comprising as component (c2) at least one additive,
(iii) mixing component (a2), the solution of component (b) according to step (i), and the dispersion of component (a1) according to step (ii) with an aqueous solution comprising as component (c3) at least one additive,

wherein the additives according to components (c1), (c2) and (c3) may be identical or different.

Preferably, the solution of component (b) is not added as the final ingredient, when mixing component (a2), the solution of component (b) and the dispersion of component (a2) with an aqueous solution comprising as component (c3) at least one additive, as described in step (iii).

As used herein, components (c1), (c2) and (c3) preferably individually represent at least one additive according to the definition of component (c). More preferably, components (c1), (c2) and (c3) individually represent at least one additive selected from the group consisting of buffering agents, osmolality adjusters, and pH adjusters. The additives according to components (c1), (c2), and (c3) may be identical or different. For example, component (c1) and (c3) may be identical, and component (c2) may be different from component (c1) and (c3).

In one embodiment, components (c1), (c2) and (c3) individually represent at least

- a buffering agent selected from the group consisting of disodium hydrogen phosphate dihydrate, sodium dihydrogen phosphate dihydrate and any mixture thereof.

Preferably, components (c1), (c2) and (c3) individually represent at least

- a buffering agent selected from the group consisting of disodium hydrogen phosphate dihydrate, sodium dihydrogen phosphate dihydrate and any mixture thereof, and
- sodium chloride as an osmolality adjuster.

More preferably, components (c1), (c2) and (c3) individually represent at least

- a buffering agent selected from the group consisting of disodium hydrogen phosphate dihydrate, sodium dihydrogen phosphate dihydrate and any mixture thereof,
- sodium chloride as an osmolality adjuster,
- sodium hydroxide or hydrogen chloride as pH adjuster.

In one embodiment, components (c1), (c2) and (c3) individually represent at least

- a mixture of disodium phosphate dihydrate and sodium dihydrogen phosphate dihydrate, and
- sodium chloride.

Preferably, components (c1), (c2) and (c3) individually represent at least

- a mixture of disodium hydrogen phosphate dihydrate and sodium dihydrogen phosphate dihydrate,
- sodium chloride, and
- an aqueous solution comprising either 1±0.5% by weight of sodium hydroxide or 1±0.5% by weight of hydrogen chloride.

In one embodiment, components (c1), (c2) and (c3) individually represent at least

- a mixture of disodium hydrogen phosphate dihydrate and sodium dihydrogen phosphate dihydrate, and
- sodium chloride

in a ratio of (2±1.5) : (10±2), preferably (1±0.5) : (10±1), more preferably (1±0,2) : (9±0.3), based on the weight of the additives.

Aqueous solutions comprising as components (c1), (c2) or (c3) at least one additive may be prepared as described in the following.

An aqueous solution comprising as component (c1) at least one additive, preferably comprises component (c1) in an amount of 3.1±2.55% by weight, preferably 1.5±0.95% by weight, more preferably 1.0±0.45% by weight based on the total weight of the solution, wherein component (c1) preferably represents at least one additive selected from the group consisting of buffering agents, osmolality adjusters, and pH adjusters.

An aqueous solution comprising as component (c2) at least one additive, preferably comprises component (c2) in an amount of 3.1±2.55% by weight, preferably 1.5±0.95% by weight, more preferably 1.0±0.45% by weight based on the total weight of the solution, wherein component (c2) preferably represents at least one additive selected from the group consisting of buffering agents, osmolality adjusters, and pH adjusters.

An aqueous solution comprising as component (c3) at least one additive, preferably comprises component (c3) in an amount of 3.1±2.55% by weight, preferably 1.5±0.95% by weight, more preferably 1.0±0.45% by weight based on the total weight of the solution, wherein component (c3) preferably represents at least one additive selected from the group consisting of buffering agents, osmolality adjusters, and pH adjusters.

Certain embodiments of aqueous solutions comprising as component (c1), (c2) or (c3) at least a buffering agent, an osmolality adjuster, optionally a pH adjuster, and optionally additional additives, may be prepared according to one of the following quantity specifications E14 to E17, wherein the wt.-% are based on the total weight of the aqueous solutions:

| | E14 | E15 | E16 | E17 |
|---|---|---|---|---|
| water | ≥ 98 wt.-% | 99±0.4 wt.-% | 99±0.2 wt.-% | 99.097 wt.-% |

(continued)

| | E14 | E15 | E16 | E17 |
|---|---|---|---|---|
| buffering agent | 0.1 ±0.05 wt.-% | 0.08±0.02 wt.-% | 0.08±0.01 wt.-% | 0.083 wt.-% |
| osmolality adjuster | 1.0±0.5 wt.-% | 0.8±0.2 wt.-% | 0.8±0.1 wt.-% | 0.82 wt.-% |
| pH adjuster | 1.0±1.0 wt.-% | 0.1±0.1 wt.-% | 0.1±0.1 wt.-% | 0 wt.-% |
| additional additives | 1.0±1.0 wt.-% | 0.1±0.1 wt.-% | 0 wt.-% | 0 wt.-% |

In other embodiments aqueous solutions comprising as component (c1), (c2) or (c3) at least disodium hydrogen phosphate dihydrate, sodium dihydrogen phosphate dihydrate, and sodium chloride, and optionally a 1 wt.-%sodium hydroxide solution or a 1 wt.-% hydrogen chloride solution, may be prepared according to one of the following quantity specifications E18 to E21, wherein the wt.-% are based on the total weight of the aqueous solutions:

| | E18 | E19 | E20 | E21 |
|---|---|---|---|---|
| water | ≥ 98 wt.-% | 99±0.4 wt.-% | 99±0.2 wt.-% | ≥ 98 wt.-% |
| disodium hydrogen phosphate dihydrate | 0.04±0.02 wt.-% | 0.04±0.01 wt.-% | 0.04±0.01 wt.-% | 0.14±0.02 wt.-% |
| sodium dihydrogen phosphate dihydrate | 0.04±0.02 wt.-% | 0.04±0.01 wt.-% | 0.04±0.01 wt.-% | 0.03±0.02 wt.-% |
| sodium chloride | 0.8±0.3 wt.-% | 0.8±0.2 wt.-% | 0.8±0.1 wt.-% | 0.8±0.3 wt.-% |
| 1 wt.-% sodium hydroxide solution, or 1 wt% hydrogen chloride solution | 0.5±0.5 wt.-% | 0.1±0.1 wt.-% | 0.1±0.1 wt.-% | 0.5±0.5 wt.-% |

In a preferred embodiment, the composition of matter is obtainable by

(i) dissolving component (b) in an amount of 6±2% by weight in an aqueous solution comprising 3.1±2.55% by weight of component (c1),
(ii) dispersing component (a1) in an amount of 2±1.5% by weight in an aqueous solution comprising 3.1±2.55% by weight of component (c2),
(iii) mixing component (a2), the solution of component (b) according to step (i), and the dispersion of component (a1) according to step (ii) with an aqueous solution comprising 3.1±2.55% by weight of component (c3), wherein the solution of component (b) is not added as the final ingredient,

wherein component (a2), the dispersion of component (a1) according to step (ii) and the solution of component (b) according to step (i) are used in amounts that

- component (a2) is contained in an amount of 0.01 to 10% by weight in the resulting composition of matter, wherein the wt.-% is based on the total weight of the composition of matter,
- component (a1) is contained in an amount of 0.001 to 5% by weight in the resulting composition of matter, wherein the wt.-% is based on the total weight of the composition of matter, and
- component (b) is contained in an amount of 0.001 to 5% by weight in the resulting composition of matter, wherein the wt.-% is based on the total weight of the composition of matter,

wherein components (c1), (c2) and (c3) represent at least one additive as defined above, and may be identical or different.

As used herein and in the following preferred embodiments, the amounts of components (c1), (b), (c2), (a1) and (c3) in wt.-% according to steps (i), (ii) and (iii) are to be understood as follows:

- the wt.-% of component (c1), which is comprised in the aqueous solution comprising (c1) is based on the total weight of the aqueous solution comprising component (c1),
- the wt.-% of component (b), which is dissolved in said aqueous solution comprising a certain amount of component (c1), according to step (i), is based on the total weight of the composition comprising component (c1) and component (b),

- the wt.-% of component (c2), which is comprised in the aqueous solution comprising component (c2) is based on the total weight of the aqueous solution comprising component (c2),
- the wt.-% of component (a1), which is dispersed in said aqueous solution comprising a certain amount of component (c2), according to step (ii), is based on the total weight of the composition comprising component (c2) and component (a1),
- the wt.-% of component (c3), which is comprised in the aqueous solution comprising (c3) is based on the total weight of the aqueous solution comprising component (c3).

Preferably, the composition of matter, which additionally comprises component (a2), is obtainable by

(i) dissolving component (b) in an amount of 6±1% by weight in an aqueous solution comprising 1±0.45% by weight of component (c1),
(ii) dispersing component (a1) in an amount of 2±0.5% by weight in an aqueous solution comprising 1±0.45% by weight of component (c2),
(iii) mixing component (a2), the solution of component (b) according to step (i), and the dispersion of component (a1) according to step (ii) with an aqueous solution comprising 1±0.45% by weight of component (c3), wherein the solution of component (b) is not added as the final ingredient,

wherein component (a2) the dispersion of component (a1) according to step (ii) and the solution of component (b) according to step (i) are used in amounts that

- component (a2) is contained in an amount of 0.5 to 2.5% by weight in the resulting composition of matter, wherein the wt.-% is based on the total weight of the composition of matter,
- component (a1) is contained in an amount of 0.01 to 0.5% by weight in the resulting composition of matter, wherein the wt.-% is based on the total weight of the composition of matter, and
- component (b) is contained in an amount of 0.1 to 1% by weight in the resulting composition of matter, wherein the wt.-% is based on the total weight of the composition of matter,

wherein components (c1), (c2) and (c3) represent at least one additive as defined above, and may be identical or different.

More preferably, the composition of matter is obtainable by

(i) dissolving component (b) in an amount of 6±1% by weight in an aqueous solution comprising 1±0.45% by weight of component (c1),
(ii) dispersing component (a1) in an amount of 2±0.5% by weight in an aqueous solution comprising 1±0.45% by weight of component (c2),
(iii) mixing component (a2), the solution of component (b) according to step (i), and the dispersion of component (a1) according to step (ii) with an aqueous solution comprising 1±0.45% by weight of component (c3), wherein the solution of component (b) is not added as the final ingredient,
(iv) adjusting the pH value to 7.0-7.2, the osmolality to 290-330 mOsmol/l, and the dynamic viscosity to 8000-16000mPa*s, and
(v) sterilizing the composition,

wherein component (a2), the dispersion of component (a1) according to step (ii) and the solution of component (b) according to step (i) are used in amounts that

- component (a2) is contained in an amount of 0.5 to 2.5% by weight in the resulting composition of matter, wherein the wt.-% is based on the total weight of the composition of matter,
- component (a1) is contained in an amount of 0.01 to 0.5% by weight in the resulting composition of matter, wherein the wt.-% is based on the total weight of the composition of matter, and
- component (b) is contained in an amount of 0.1 to 1% by weight in the resulting composition of matter, wherein the wt.-% is based on the total weight of the composition of matter,

wherein components (c1), (c2) and (c3) represent at least one additive as defined above, and may be identical or different.

Preferably, step (iv) is performed by adding one or more solutions selected from the group consisting of

- an aqueous solution comprising 0.05 to 0.5% by weight, preferably 0.05 to 0.2% by weight, of disodium phosphate dihydrate and/or sodium dihydrogen phosphate dihydrate,
- an aqueous solution comprising 0.5 to 5% by weight, preferably 0.8 to 1 % by weight, of sodium chloride,

- an aqueous solution comprising either 1±0.5% by weight of sodium hydroxide or 1±0.5% by weight of hydrogen chloride,
- an aqueous solution comprising 1±0.5% by weight of a thickening agent, such as arabic gums and their derivatives, lambda carrageenan, and pullulan gums and their derivatives.

Preferably, step (v) is performed at a temperature of 60-140°C, more preferably 100-125°C, most preferably 115-122°C.

In a further aspect, the present invention is directed to a method of preparing the composition of matter comprising

(i) dissolving component (b) in an aqueous solution comprising as component (c1) at least one additive,
(ii) dispersing component (a1) in an aqueous solution comprising as component (c2) at least one additive,
(iii) mixing component (a2), the solution of component (b) according to step (i), and the dispersion of component (a1) according to step (ii) with an aqueous solution comprising as component (c3) at least one additive,

wherein the additives according to components (c1), (c2) and (c3) may be identical or different.

Preferably, the solution of component (b) is not added as the final ingredient, when mixing component (a2), the solution of component (b) and the dispersion of component (a2) with an aqueous solution comprising as component (c3) at least one additive, as described in step (iii).

As used herein and in the following preferred embodiments, components (c1), (c2) and (c3) preferably individually represent at least one additive as defined above. Aqueous solutions comprising as components (c1), (c2) or (c3) at least one additive may also be prepared as described above.

In a preferred embodiment, the method of preparing the composition of matter comprises

(i) dissolving component (b) in an amount of 6±2% by weight in an aqueous solution comprising 3.1±2.55% by weight of component (c1),
(ii) dispersing component (a1) in an amount of 2±1.5% by weight in an aqueous solution comprising 3.1±2.55% by weight of component (c2),
(iii) mixing component (a2), the solution of component (b) according to step (i), and the dispersion of component (a1) according to step (ii) with an aqueous solution comprising 3.1±2.55% by weight of component (c3), wherein the solution of component (b) is not added as the final ingredient,

wherein component (a2), the dispersion of component (a1) according to step (ii) and the solution of component (b) according to step (i) are used in amounts that

- component (a2) is contained in an amount of 0.01 to 10% by weight in the resulting composition of matter, wherein the wt.-% is based on the total weight of the composition of matter,
- component (a1) is contained in an amount of 0.001 to 5% by weight in the resulting composition of matter, wherein the wt.-% is based on the total weight of the composition of matter, and
- component (b) is contained in an amount of 0.001 to 5% by weight in the resulting composition of matter, wherein the wt.-% is based on the total weight of the composition of matter,

and wherein components (c1), (c2) and (c3) represent at least one additive as defined above, and may be identical or different.

As used herein and in the following preferred embodiments, which relate to "the method of preparing the composition of matter", the amounts of components (c1), (b), (c2), (a1) and (c3) in wt.-% according to steps (i), (ii) and (iii) are to be understood as follows:

- the wt.-% of component (c1), which is comprised in the aqueous solution comprising (c1) is based on the total weight of the aqueous solution comprising component (c1),
- the wt.-% of component (b), which is dissolved in said aqueous solution comprising a certain amount of component (c1), according to step (i), is based on the total weight of the composition comprising component (c1) and component (b),
- the wt.-% of component (c2), which is comprised in the aqueous solution comprising component (c2) is based on the total weight of the aqueous solution comprising component (c2),
- the wt.-% of component (a1), which is dispersed in said aqueous solution comprising a certain amount of component (c2), according to step (ii), is based on the total weight of the composition comprising component (c2) and component (a1),
- the wt.-% of component (c3), which is comprised in the aqueous solution comprising (c3) is based on the total weight

of the aqueous solution comprising component (c3).

Preferably, the method of preparing the composition of matter comprises

(i) dissolving component (b) in an amount of 6±1% by weight in an aqueous solution comprising 1±0.45% by weight of component (c1),
(ii) dispersing component (a1) in an amount of 2±0.5% by weight in an aqueous solution comprising 1±0.45% by weight of component (c2),
(iii) mixing component (a2), the solution of component (b) according to step (i), and the dispersion of component (a1) according to step (ii) with an aqueous solution comprising 1±0.45% by weight of component (c3), wherein the solution of component (b) is not added as the final ingredient,

wherein component (a2), the dispersion of component (a1) according to step (ii) and the solution of component (b) according to step (i) are used in amounts that

- component (a2) is contained in an amount of 0.5 to 2.5% by weight in the resulting composition of matter, wherein the wt.-% is based on the total weight of the composition of matter,
- component (a1) is contained in an amount of 0.01 to 0.5% by weight in the resulting composition of matter, wherein the wt.-% is based on the total weight of the composition of matter, and
- component (b) is contained in an amount of 0.1 to 1% by weight in the resulting composition of matter, wherein the wt.-% is based on the total weight of the composition of matter,

and wherein components (c1), (c2) and (c3) represent at least one additive as defined above, and may be identical or different.

More preferably, the method of preparing the composition of matter comprises

(i) dissolving component (b) in an amount of 6±1% by weight in an aqueous solution comprising 1±0.45% by weight of component (c1),
(ii) dispersing component (a1) in an amount of 2±0.5% by weight in an aqueous solution comprising 1±0.45% by weight of component (c2),
(iii) mixing component (a2), the solution of component (b) according to step (i), and the dispersion of component (a1) according to step (ii) with an aqueous solution comprising 1±0.45% by weight of component (c3), wherein the solution of component (b) is not added as the final ingredient,
(iv) adjusting the pH value to 7.0-7.2, the osmolality to 290-330 mOsmol/l, and the dynamic viscosity to 8000-16000mPa*s, and
(v) sterilizing the composition,

wherein component (a2), the dispersion of component (a1) according to step (ii) and the solution of component (b) according to step (i) are used in amounts that

- component (a2) is contained in an amount of 0.5 to 2.5% by weight in the resulting composition of matter, wherein the wt.-% is based on the total weight of the composition of matter,
- component (a1) is contained in an amount of 0.01 to 0.5% by weight in the resulting composition of matter, wherein the wt.-% is based on the total weight of the composition of matter, and
- component (b) is contained in an amount of 0.1 to 1% by weight in the resulting composition of matter, wherein the wt.-% is based on the total weight of the composition of matter,

and wherein components (c1), (c2) and (c3) represent at least one additive as defined above, and may be identical or different.

Preferably, step (iv) is performed by adding one or more solutions selected from the group consisting of

- an aqueous solution comprising 0.05 to 0.5% by weight, preferably 0.05 to 0.2% by weight, of disodium phosphate dihydrate and/or sodium dihydrogen phosphate dihydrate,
- an aqueous solution comprising 0.5 to 5% by weight, preferably 0.8 to 1% by weight, of sodium chloride,
- an aqueous solution comprising either 1±0.5% by weight of sodium hydroxide or 1±0.5% by weight of hydrogen chloride,
- an aqueous solution comprising 1±0.5% by weight of a thickening agent, such as arabic gums and their derivatives, lambda carrageenan, and pullulan gums and their derivatives.

Preferably, step (v) is performed at a temperature of 60-140°C, more preferably 100-125°C, most preferably 115-122°C.

In a further aspect, the invention is directed to the composition of matter for use

(I) in the treatment of skin and/or mucous membrane changes,
(II) in cosmetic surgery, and/or
(III) in ophthalmologic surgery

In a preferred embodiment, the composition of matter is useful in cosmetic surgery.

Preferably, the composition of matter is useful for volume replacements, such as refilling of folds and lip augmentation.

In one embodiment, the composition of matter is administered by injection, preferably by subcutaneous injection.

If administered by injection, the composition of matter is particularly useful

(I) in the treatment of skin and/or mucous membrane changes,
(II) in cosmetic surgery, and/or
(III) in ophthalmologic surgery

and in the simultaneous treatment and/or prevention of pain caused by the injection of the composition. Preferably, also foreign body reactions caused by the injection can simultaneously be prevented and/or treated.

The composition of matter preferably comprises component (a1) and component (a2) for the replacement of hyaluronic acid in the skin.

The composition of matter preferably comprises component (b) as an active ingredient for the treatment and/or prevention of pain caused by the injection of the composition. Component (b) is also useful as an active ingredient for the treatment and/or prevention of acute inflammation and injection-induced foreign body reactions.

In one embodiment, the composition, which comprises as component (b) prilocaine hydrochloride, is administered in an amount which corresponds to a dose of up to 12 mg prilocaine hydrochloride, preferably 0.3 to 9 mg prilocaine hydrochloride, more preferably 3 to 6 mg prilocaine hydrochloride. In a preferred embodiment, the composition may be administered by one injection, which may correspond, for example, to a dose of $3\pm1$ mg or $6\pm1$ mg prilocaine hydrochloride. Preferably, the volume of the injections of the composition of matter can be varied by reducing or increasing the amount of components (a1), (a2) and optionally (c), but not changing the amount of component (b). It is particularly preferable to administer a volume of 0.1 to 4 ml, preferably 0.2 to 3 ml per injection in one injection, wherein the prilocaine hydrochloride dose is 0.3 to 9 mg, preferably 3 to 6 mg.

In one embodiment, the composition of matter may be administered to the patient at least once a year, preferably twice a year. Depending on the intended use and individual factors residing in the patient, the frequency of administration may be adjusted, for example, up to one administration per month.

In one embodiment, 0.1 to 1 ml of the composition of matter comprising $0.1\pm0.05$ wt.-% of component (a1), $1.4\pm0.6$ wt.-% of component (a2), and $0.3\pm0.1$ wt.-%, in each cased based on the total weight of the composition, are administered by subcutaneous injection. Preferably, 0.1 to 1 ml of the composition of matter comprising as component (a1) $0.1\pm0.05$ wt.-% of sodium hyaluronate with a molecular weight of 2,500,000 to 3,500,000 Dalton, as component (a2) $1.4\pm0.6$ wt.-% of BDDE-cross-linked sodium hyaluronate with a molecular weight of 900,000 to 1,100,000 Dalton and with a cross-linking degree of 42 to 55%, and as component (b) $0.3\pm0.1$ wt.-% prilocaine hydrochloride, in each case based on the total weight of the composition, are administered by subcutaneous injection. If, for example, 0.1 ml of the composition of matter is administered, this corresponds to a dose of $0.1\pm0.05$ mg of the sodium hyaluronate, a dose of $1.4\pm0.6$ mg of the BDDE-cross-linked sodium hyaluronate, and a dose of $0.3\pm0.1$ mg of the prilocaine hydrochloride. If 1 ml of the composition is administered, this corresponds to a dose of $1\pm0.5$ mg mg of the sodium hyaluronate, a dose of $14\pm6$ mg mg of the BDDE-cross-linked sodium hyaluronate and a dose of $3\pm1$ mg of the prilocaine hydrochloride. Accordingly, the administration of a dose of 0.05 to 1.5 mg of sodium hyaluronate (2,500,000 to 3,500,000 Dalton), a dose of 0.8 to 20 mg of BDDE-cross-linked sodium hyaluronate (900,000 to 1,100,000 Dalton, cross-linking degree = 42 to 55%) and a dose of 0.2 to 4 mg of prilocaine hydrocloride is particularly preferred, if the composition of matter is administered by subcutaneous injection.

In one embodiment, component (b) is released according to the in vitro dissolution profile as indicated in table A above.

In another embodiment, component (b) is released in an amount of at least 20% after 10 minutes, at least 50% after 45 minutes, and at least 80% after 120 minutes after the injection. Preferably, component (b) is released in an amount of at least 20% after 10 minutes, at least 60% after 45 minutes, and at least 85% after 120 minutes. As used herein, the values in % are preferably to be understood as wt.-% based in the total weight of component (b) in the composition of matter.

The release of component (b) in the inventively claimed compositions of matter may be determined by the in-vitro-dissolution profile. A method was developed to measure the dissolution characteristics using tubes for dialysis as cavities. The following parameters are preferably used:

| | |
|---|---|
| Instrument: | Dissolution tester Sotax AT 7 smart or comparable |
| Sample: | 1 g in 9 cm dialyse tube Spectra/Por |
| Device: | Paddle |
| Rate: | 75 rpm |
| Medium: | Buffer solution pH = 6.8 R; containing $Na_2HPO_4$ x 12 $H_2O$ dissolved in water R (71.5 g/L) and Citric acid x 1 $H_2O$ dissolved in water R (21 g/L) and 500 mL |
| Volume: | |
| Temperature: | 37°C±0.5°C |
| Removal volume: | 2.5 mL |
| Profile [min]: | 10/45/120 for routine testing |

The HPLC method for detection of component (b) is for example possible with UV detection at 230 nm according to the method of external standard. The dissolution profiles are typically established with 8 time points. The pH of the dissolution medium used as acceptor compartment is 6.8 corresponding to the physiological pH.

As used herein, the release of component (b) in a certain amount after a certain time corresponds to the in vitro dissolution profile.

In a further aspect, the invention is directed to the composition of matter for use

(IV) in the treatment of degenerative joint diseases including osteoarthritis and/or traumatic joint diseases,
(V) in the treatment of articular cartilage and/or cartilage bone defects,
(VI) in the treatment of meniscus lesions and/or intervertebral disc lesions,
(VII) in the treatment of arthrosis and/or articular rheumatism, and/or
(VIII) in the treatment of osteochondritis dissecans and/or flake fractures.

In one embodiment, the composition of matter is administered by injection, preferably by intra-articular injection.

In a preferred embodiment, the composition of matter is administered by injection into a knee, a shoulder, or a hip. The administration in a knee is particularly preferred.

If administered by intra-articular injection, the composition of matter is particularly useful

(IV) in the treatment of degenerative joint diseases including osteoarthritis and/or traumatic joint diseases,
(V) in the treatment of articular cartilage and/or cartilage bone defects,
(VI) in the treatment of meniscus lesions and/or intervertebral disc lesions,
(VII) in the treatment of arthrosis and/or articular rheumatism, and/or
(VIII) in the treatment of osteochondritis dissecans and/or flake fractures,

and in the simultaneous treatment and/or prevention of pain caused by the intra-articular injection of the composition. Preferably, also foreign body reactions caused by the intra-articular injection can simultaneously be prevented and/or reduced.

The composition of matter preferably comprises component (b) as an active ingredient for the treatment and/or prevention of pain caused by the intra-articular injection of the composition. Component (b) is also useful as an active ingredient for the treatment and/or prevention of acute inflammation and injection-induced foreign body reactions.

In one embodiment, the composition, which comprises as component (b) prilocaine hydrochloride, is administered in an amount which corresponds to a dose of up to 24 mg prilocaine hydrochloride, preferably 3 to 18 mg prilocaine hydrochloride more preferably 3 to 12 mg prilocaine hydrochloride, most preferably 3 to 6 mg prilocaine hydrochloride. In a preferred embodiment, the composition may be administered by one injection, which corresponds to a dose of 3±1 mg or 6±1 mg prilocaine hydrochloride. Preferably, the volume of the injections of the composition of matter can be varied by reducing or increasing the amount of components (a1), (a2) and optionally (c), but not changing the amount of component (b). It is particularly preferable to administer a volume of 1 to 8 ml, preferably 2 to 8 ml or 2 to 6 per injection in one injection, wherein the prilocaine hydrochloride dose is 3 to 24 mg.

In one embodiment, the composition of matter may be administered to the patient at least once a year, preferably twice a year. Depending on the intended use and individual factors residing in the patient, the frequency of administration may be adjusted, for example, between up to one administration per month or one administration in a two-year-period.

In one embodiment, 4 to 8 ml of the composition of matter comprising 0.1±0.05 wt.-% of component (a1), 1.4±0.6 wt.-% of component (a2), and 0.3±0.1 wt.-%, in each cased based on the total weight of the composition, are administered by intra-articular injection. Preferably, 4 to 8 ml of the composition of matter, comprising as component (a1) 0.1±0.05 wt.-% of sodium hyaluronate with a molecular weight of 2,500,000 to 3,500,000 Dalton, as component (a2) 1.4±0.6 wt.-% of BDDE-cross-linked sodium hyaluronate with a molecular weight of 900,000 to 1,100,000 Dalton and with a cross-linking degree of 42 to 55%, and as component (b) 0.3±0.1 wt.-% prilocaine hydrochloride, in each case based on the total weight of the composition, are administered by intra-articular injection. Preferably, the BDDE-cross-linked sodium hyaluronate in the composition of matter has a particle size distribution characterized in that at least 95 vol.-% of the particles have a diameter lower than 300 μm, at least 90 vol.-% of the particles have a diameter lower than 250 μm, at least 50 vol.-% of the particles have a diameter lower than 120 μm, at least 10 vol.-% of the particles have a diameter lower than 50 μm, at least 5 vol.-% of the particles have a diameter lower than 45 μm, wherein the vol.-% are based on the total volume of the BDDE-cross-linked sodium hyaluronate in the composition.

If, for example, 4 ml of the composition of matter are administered, this corresponds to a dose of 4±2 mg of the sodium hyaluronate, a dose of 56±24 mg of the BDDE-cross-linked sodium hyaluronate, and a dose of 12±4 mg of the prilocaine hydrochloride. If 8 ml of the composition are administered, this corresponds to a dose of 8±4 mg of the sodium hyaluronate, a dose of 112±48 mg of the BDDE-cross-linked sodium hyaluronate and a dose of 24±8 mg of the prilocaine hydrochloride. Accordingly, the administration of a dose of 2 to 12 mg of sodium hyaluronate (2,500,000 to 3,500,000 Dalton), a dose of 48 to 160 mg of BDDE-cross-linked sodium hyaluronate (900,000 to 1,100,000 Dalton, cross-linking degree = 42 to 55%) and a dose of 8 to 32 mg of prilocaine hydrocloride is particularly preferred, if the composition of matter is administered by intra-articular injection.

In one embodiment, the composition of matter is administered by intra-articular injection only once or more than once. If the composition of matter is administered more than once, it may be administered on a regular basis at least once in a year, preferably every eighth month, every sixth month or every fourth month. Optionally, the dose can be reduced after the initial administration. For a subsequent administration, a dose of components (a1), (a2) and (b) can be sufficient, which preferably corresponds to 75% or 50% by weight of the dose of components (a1), (a2) and (b), which has been administered in the initial administration. For example, if a composition of matter has been administered in an amount corresponding to a dose of 12 mg prilocaine hydrochloride in the initial treatment, a reduced amount corresponding to a dose of 9 or 6 mg prilocaine hydrochloride may be used in the subsequent treatment.

In one embodiment, 2 to 8 ml, preferably 2 to 6 ml of the inventive composition of matter are administered by one injection. The amount to be administered may be split up and may be given to the patient in separate treatment session.

In one embodiment, component (b) is released according to the in vitro dissolution profile as indicated in table A above.

In another embodiment, component (b) is released in an amount of at least 20% after 10 minutes, at least 50% after 45 minutes, and at least 80% after 120 minutes. Preferably, component (b) is released in an amount of at least 20% after 10 minutes, at least 60% after 45 minutes, and at least 85% after 120 minutes. As used herein, the values in % are preferably to be understood as wt.-% based in the total weight of component (b) in the composition of matter. Furthermore, the release of component (b) in a certain amount after a certain time corresponds to the in vitro dissolution profile, which may be determined as indicated above.

In a further aspect, the invention is directed to the composition of matter for use

(IX) in the treatment of osteoarthritis,
(X) in the treatment and/or prevention of acute inflammation in a subject suffering from osteoarthritis,
(XI) in the treatment of osteoarthritis and simultaneous treatment and/or prevention of acute inflammation,
(XII) in the treatment and/or prevention of pain in a subject suffering from osteoarthritis, and/or
(XIII) in the treatment of osteoarthritis and simultaneous treatment and/or prevention of pain.

In one embodiment, the composition of matter is administered by injection, preferably by intra-articular injection.

In a preferred embodiment, the composition of matter is administered by injection into a knee, a shoulder, or a hip. The administration in a knee is particularly preferred.

If administered by intra-articular injection, the composition of matter is particularly useful

(IX) in the treatment of osteoarthritis,
(X) in the treatment and/or prevention of acute inflammation in a subject suffering from osteoarthritis,
(XI) in the treatment of osteoarthritis and simultaneous treatment and/or prevention of acute inflammation,
(XII) in the treatment and/or prevention of pain in a subject suffering from osteoarthritis, and/or
(XIII) in the treatment of osteoarthritis and simultaneous treatment and/or prevention of pain,

and in the simultaneous treatment and/or prevention of pain caused by the intra-articular injection of the composition.

Preferably, also foreign body reactions caused by the intra-articular injection can simultaneously be prevented and/or reduced.

The composition of matter comprises component (b) as an active ingredient for the treatment and/or prevention of pain caused by the intra-articular injection of the composition.

In one embodiment, the composition, which comprises as component (b) prilocaine hydrochloride, is administered in an amount which corresponds to a dose of up to 24 mg prilocaine hydrochloride, preferably 3 to 18 mg prilocaine hydrochloride more preferably 3 to 12 mg prilocaine hydrochloride, most preferably 3 to 6 mg prilocaine hydrochloride. In a preferred embodiment, the composition may be administered by one injection, which corresponds to a dose of 3±1 mg or 6±1 mg prilocaine hydrochloride. Preferably, the volume of the injections of the composition of matter can be varied by reducing or increasing the amount of components (a1), (a2) and optionally (c), but not changing the amount of component (b). It is particularly preferable to administer a volume of 1 to 8 ml, preferably 2 to 8 ml or 2 to 6 per injection in one injection, wherein the prilocaine hydrochloride dose is 3 to 24 mg.

In one embodiment, the composition of matter may be administered to the patient at least once a year, preferably twice a year. Depending on the intended use and individual factors residing in the patient, the frequency of administration may be adjusted, for example, between up to one administration per month or one administration in a two-year-period.

In one embodiment, 4 to 8 ml of the composition of matter comprising 0.1±0.05 wt.-% of component (a1), 1.4±0.6 wt.-% of component (a2), and 0.3±0.1 wt.-%, in each cased based on the total weight of the composition, are administered by intra-articular injection. Preferably, 4 to 8 ml of the composition of matter, comprising as component (a1) 0.1±0.05 wt.-% of sodium hyaluronate with a molecular weight of 2,500,000 to 3,500,000 Dalton, as component (a2) 1.4±0.6 wt.-% of BDDE-cross-linked sodium hyaluronate with a molecular weight of 900,000 to 1,100,000 Dalton and with a cross-linking degree of 42 to 55%, and as component (b) 0.3±0.1 wt.-% prilocaine hydrochloride, in each case based on the total weight of the composition, are administered by intra-articular injection. Preferably, the BDDE-cross-linked sodium hyaluronate in the composition of matter has a particle size distribution characterized in that at least 95 vol.-% of the particles have a diameter lower than 300 μm, at least 90 vol.-% of the particles have a diameter lower than 250 μm, at least 50 vol.-% of the particles have a diameter lower than 120 μm, at least 10 vol.-% of the particles have a diameter lower than 50 μm, at least 5 vol.-% of the particles have a diameter lower than 45 μm, wherein the vol.-% are based on the total volume of the BDDE-cross-linked sodium hyaluronate in the composition.

If, for example, 4 ml of the composition of matter are administered, this corresponds to a dose of 4±2 mg of the sodium hyaluronate, a dose of 56±24 mg of the BDDE-cross-linked sodium hyaluronate, and a dose of 12±4 mg of the prilocaine hydrochloride. If 8 ml of the composition are administered, this corresponds to a dose of 8±4 mg of the sodium hyaluronate, a dose of 112±48 mg of the BDDE-cross-linked sodium hyaluronate and a dose of 24±8 mg of the prilocaine hydrochloride. Accordingly, the administration of a dose of 2 to 12 mg of sodium hyaluronate (2,500,000 to 3,500,000 Dalton), a dose of 48 to 160 mg of BDDE-cross-linked sodium hyaluronate (900,000 to 1,100,000 Dalton, cross-linking degree = 42 to 55%) and a dose of 8 to 32 mg of prilocaine hydrocloride is particularly preferred, if the composition of matter is administered by intra-articular injection.

In one embodiment, the composition of matter is administered by intra-articular injection only once or more than once. If the composition of matter is administered more than once, it may be administered on a regular basis at least once in a year, preferably every eighth month, every sixth month or every fourth month. Optionally, the dose can be reduced after the initial administration. For a subsequent administration, a dose of components (a1), (a2) and (b) can be sufficient, which preferably corresponds to 75% or 50% by weight of the dose of components (a1), (a2) and (b), which has been administered in the initial administration. For example, if a composition of matter has been administered in an amount corresponding to a dose of 12 mg prilocaine hydrochloride in the initial treatment, a reduced amount corresponding to a dose of 9 or 6 mg prilocaine hydrochloride may be used in the subsequent treatment.

In one embodiment, 4 to 8 ml, preferably 4 to 6 ml of the composition of matter are administered by one injection.

In another embodiment, 4 to 8 ml, preferably 6 to 8 ml of the composition of matter are administered by more than one injection, preferably by four injections of 1, 1.5 or 2 ml. The 4 injections may be carried out at once, optionally in different positions of the joint, or on different days within a time period of two weeks.

In one embodiment, component (b) is released according to the in vitro dissolution profile as indicated in table A above.

In another embodiment, component (b) is released in an amount of at least 20% after 10 minutes, at least 50% after 45 minutes, and at least 80% after 120 minutes. Preferably, component (b) is released in an amount of at least 20% after 10 minutes, at least 60% after 45 minutes, and at least 85% after 120 minutes. As used herein, the values in % are preferably to be understood as wt.-% based in the total weight of component (b) in the composition of matter. Furthermore, the release of component (b) in a certain amount after a certain time corresponds to the in vitro dissolution profile, which may be determined as indicated above.

Figures 1 to 7 show the in vitro dissolution characteristics of prilocaine hydrochloride in inventively claimed compositions A, B and C. Figures 8 and 9 show the in vitro dissolution characteristics of composition X, which is a commercially available solution of prilocaine not comprising hyaluronic acid and/or a pharmaceutically acceptable salt

thereof.

Figure 1: Composition A: Release (in-vitro-dissolution) of prilocaine hydrochloride, precision (n=6), paddle, 75 rpm
Figure 2: Composition A: Release (in-vitro-dissolution) of prilocaine hydrochloride, intermediate precision (n=6), paddle, 75 rpm
Figure 3: Composition B: Release (in-vitro-dissolution) of prilocaine hydrochloride, precision (n=6), paddle, 75 rpm
Figure 4: Composition B: Release (in-vitro-dissolution) of prilocaine hydrochloride, intermediate precision (n=6), paddle, 75 rpm
Figure 5: Composition C (Sample 1): Release (in-vitro-dissolution) of prilocaine hydrochloride, precision (n=6), paddle, 75 rpm
Figure 6: Composition C (Sample 1): Release (in-vitro-dissolution) of prilocaine hydrochloride, intermediate precision (n=6), paddle, 75 rpm
Figure 7: Composition C (Sample 2): Release (in-vitro-dissolution) of prilocaine hydrochloride, precision (n=6), paddle, 75 rpm
Figure 8: Composition X: Release (in-vitro-dissolution) of prilocaine hydrochloride, precision (n=6), paddle, 75 rpm
Figure 9: Composition X: Release (in-vitro-dissolution) of prilocaine hydrochloride, intermediate precision (n=6), paddle, 75 rpm

The following examples further illustrate the invention, but are not to be construed as limiting its scope.

Examples:

General:

In the following examples sodium hyaluronate of non-human and non-animal origin was used, which is characterized by the following specifications (in accordance with Pharmacopoea. Europaea (Ph. Eur.) monograph N°1472):

- appearance: white or almost white fibrous aggregate, sparingly soluble to soluble in water, practically insoluble in acetone and ethanol
- absorbance 600 nm (0.33% [w/v] solution, dried): ≤ 0.010
- pH (0.5% [w/v] in water, dried): 5.0 to 8.5
- intrinsic viscosity at 25°C: 2.8 to 3.2 $m^3$/kg
- average molecular weight: 2.9 to 3.5 Mio. Dalton
- absorbance 260 nm (0.33% [w/v] solution, dried): ≤ 0.200
- protein: ≤ 0.1%
- chlorides: ≤ 0.3%
- iron: ≤ 80 ppm
- heavy metals: ≤ 10 ppm
- loss on drying: ≤ 15.0%
- total viable aerobic count: ≤ 100 CFU/g
- bacterial endotoxins (0.125% solution): < 0.05 I.U./mg
- sodium hyaluronate content (on dried): 95.0 to 105.0 %

As cross-linked sodium hyaluronate 1,4-butandiol-diglycidylether-(BDDE)-cross-linked sodium hyaluronate having a cross-linking degree of 42-55% was used.
Both, non-cross-linked as well as cross-linked sodium hyaluronate may be obtained via BioPolymer GmbH & Co. KG, Montabaur, Germany.
General properties of prilocaine hydrochloride are described in Pharmacopoea. Europaea (Ph. Eur.) monograph N°1363. Prilocaine hydrochloride as used in the examples is manufactured, for example, by BASF Orgamol Pharma Solutions SA (Switzerland) and supplied by Fagron GmbH & Co. KG (Germany).
The preparations of compositions A, B and C in examples 1-3 were performed under sterile conditions.

Example 1

A composition of matter may be prepared employing the following aqueous solutions:
Aqueous solution comprising component (c):

| | |
|---|---|
| Water | 991.0 g |
| Disodium hydrogen phosphate dihydrate | 0.44 g |
| Sodium dihydrogen phosphate dihydrate | 0.39 g |
| Sodium chloride | 8.2 g |

Starting from the aqueous solution comprising component (c) three different solutions are prepared, which differ from each other with respect to the amount of added NaOH or HCl solution for adjusting the pH value. Said aqueous solutions are specified as aqueous solutions comprising component (c1), component (c2) or component (c3) respectively, and, in the following, further characterized by their pH value.
Aqueous solution comprising component (c1), pH = 7.0:

| | |
|---|---|
| Water | 991.0 g |
| Disodium hydrogen phosphate dihydrate | 0.44 g |
| Sodium dihydrogen phosphate dihydrate | 0.39 g |
| Sodium chloride | 8.2 g |
| Sterile filtered 1 wt.-% solution of NaOH<br>Sterile filtered 1 wt.-% solution of HCl | } as required to obtain pH = 7.0 |

Aqueous solution comprising component (c2), pH = 7.4:

| | |
|---|---|
| Water | 991.0 g |
| Disodium hydrogen phosphate dihydrate | 0.44 g |
| Sodium dihydrogen phosphate dihydrate | 0.39 g |
| Sodium chloride | 8.2 g |
| Sterile filtered 1 wt.-% solution of NaOH<br>Sterile filtered 1 wt.-% solution of HCl | } as required to obtain pH = 7.4 |

Aqueous solution comprising component (c3), pH = 7.0:

| | |
|---|---|
| Water | 991.0 g |
| Disodium hydrogen phosphate dihydrate | 0.44 g |
| Sodium dihydrogen phosphate dihydrate | 0.39 g |
| Sodium chloride | 8.2 g |
| Sterile filtered 1 wt.-% solution of NaOH<br>Sterile filtered 1 wt.-% solution of HCl | } as required to obtain pH = 7.0 |

Component (b) is dissolved in the solution of component (c1). The resulting aqueous solution of component (b) (i.e. prilocaine hydrochloride) comprises:

| | |
|---|---|
| Prilocaine hydrochloride | 6.0 g |
| Aqueous solution comprising component (c1) having a pH of 7.0 | 94.0 g |
| | *100.0 g* |

Component (a1) is dispersed in the solution of component (c2). The resulting aqueous dispersion of component (a1) (i.e. sodium hyaluronate, ≥ 2.5 Mio Dalton):

| | |
|---|---|
| Sodium hyaluronate (2.9-3.5 Mio Dalton) | 2.0 g (plus water content) |
| Aqueous solution comprising component (c2) having a pH of 7.4 | 98.0 g |
| | *100.0 g* |

An inventive composition of matter is prepared by mixing component (a2), i.e. BDDE-cross-linked sodium hyaluronate (~1 Mio Dalton, cross-linking degree 42-55%), the aqueous solution of component (b), i.e. prilocaine hydrochloride in a solution of component (c1), and the aqueous dispersion of component (a1), i.e. sodium hyaluronate (2.9-3.5 Mio Dalton) in a solution of component (c2), with an aqueous solution of component (c3).

The following amounts are used for the preparation of the final inventive composition A:

| | |
|---|---|
| Component (a2) | 20 g |
| Aqueous dispersion comprising components (a1) and (c2) | 100 g |
| Aqueous solution comprising components (b) and (c1) | 100 g |
| Aqueous solution of component (c3) | 1780 g |
| | *2000.0 g* |

Thus, in detail, 1 ml of the composition A comprises

| Component | | Quality |
|---|---|---|
| Prilocaine hydrochloride | 3.0 mg | Ph. Eur. 1363[1)] |
| Sodium hyaluronate, crosslinked | 10.0 mg | Ph. Eur. 1472[1)], |
| Sodium hyaluronate | 1.0 mg | Ph. Eur. 1472[1)] |
| Sodium chloride | 9.0 mg | Ph. Eur. 0193[1)] |
| Disodium hydrogen phosphate dihydrate | 0.44 mg | Ph. Eur. 0602[1)] |
| Sodium dihydrogen phosphate dihydrate | 0.39 mg | Ph. Eur. 0194[1)] |
| Water | 976.2 mg | Ph. Eur. 0169[1)] |
| [1)] number of the current Pharmacopoea. Europaea (Ph. Eur.) monograph | | |

The composition is supplied sterile in a 1 ml syringe barrel with an integrated Luerlock adapter and tip cap, piston stopper and piston rod in a blister pack for single use only. Sterile needles and instructions for use are packaged in a carton together with the blister pack.

<u>Example 2</u>

The inventive composition B is prepared accordingly, but varying the relative amounts of the active ingredients.

1 ml of composition B comprises:

| Component | | Quality |
|---|---|---|
| Prilocaine hydrochloride | 3.0 mg | Ph. Eur. 1363[1)] |
| Sodium hyaluronate, crosslinked | 20.0 mg | Ph. Eur. 1472[1)], |
| Sodium hyaluronate | 1.0 mg | Ph. Eur. 1472[1)] |
| Sodium chloride | 9.0 mg | Ph. Eur. 0193[1)] |
| Disodium hydrogen phosphate dihydrate | 0.43 mg | Ph. Eur. 0602[1)] |
| Sodium dihydrogen phosphate dihydrate | 0.38 mg | Ph. Eur. 0194[1)] |
| Water | 966.2 mg | Ph. Eur. 0169[1)] |
| [1)] number of the current Ph. Eur. monograph | | |

Example 3

The inventive composition C is prepared accordingly, but varying the relative amounts of the active ingredients.

1 ml of composition C comprises:

| Component | | Quality |
|---|---|---|
| Prilocaine hydrochloride | 3.0 mg | Ph. Eur. 1363[1)] |
| Sodium hyaluronate, crosslinked | 8.0 mg | Ph. Eur. 1472[1)], |
| Sodium hyaluronate | 1.0 mg | Ph. Eur. 1472[1)] |
| Sodium chloride | 9.0 mg | Ph. Eur. 0193[1)] |
| Disodium hydrogen phosphate dihydrate | 1.4 mg | Ph. Eur. 0602[1)] |
| Sodium dihydrogen phosphate dihydrate | 0.26 mg | Ph. Eur. 0920[1)] |
| Water | 977.3 mg | Ph. Eur. 0169[1)] |
| [1)] number of the current Ph. Eur. monograph | | |

Example 4

It has been demonstrated that prilocaine hydrochloride is rapidly released from the gel matrix in the inventive compositions A, B and C according to examples 1-3. A method was developed to measure the dissolution characteristics using tubes for dialysis as cavities. The following parameters are used:

| | |
|---|---|
| Instrument: | Dissolution tester Sotax AT 7 smart or comparable |
| Sample: | 1 g in 9 cm dialyse tube Spectra/Por |
| Device: | Paddle |
| Rate: | 75 rpm |
| Medium: | Buffer solution pH = 6.8 R; containing $Na_2HPO_4$ x 12 $H_2O$ dissolved in water R (71.5 g/L) and Citric acid x 1 $H_2O$ dissolved in water R (21 g/L) and 500 mL |
| Volume: | |
| Temperature: | 37°C±0.5°C |
| Removal volume: | 2.5 mL |
| Profile [min]: | 10/45/120 for routine testing |

The HPLC method for detection of prilocaine is based on UV detection at 230 nm according to the method of external standard.

The dissolution profiles were established with 8 time points. The pH of the dissolution medium used as acceptor compartement was 6.8 corresponding to the physiological pH.

The following samples were tested:

Composition A
Composition B
Composition C (two samples)

The results of the tests are presented in the following tables 1 to 7. The results are also used for validation of the dissolution testing method. Therefore the tables are titled with "precision" (n=6) and "intermediate precision" (n=6). The requirements of the guideline CPMP/EWP/QWP/1401/98: "12 individual values for every time point for each formulation" is fulfilled.

Table 1:

| Composition A: Release (in-vitro-dissolution) of prilocaine hydrochloride, precision (n=6), paddle, 75 rpm | | | | | | |
|---|---|---|---|---|---|---|
| Dissolution [%] | | | | | | |
| Withdrawal [min] | Gel A1 | Gel A2 | Gel A3 | Gel A4 | Gel A5 | Gel A6 |
| 5 | 20.3 | 17.6 | 17.6 | 15.3 | 14.5 | 13.7 |
| 10 | 29.8 | 27.4 | 28.5 | 25.3 | 23.8 | 23.3 |
| 20 | 44.9 | 42.7 | 44.1 | 40.6 | 38.6 | 38.3 |
| 30 | 56.8 | 53.8 | 55.7 | 51.8 | 50.1 | 49.5 |
| 45 | 69.4 | 66.2 | 68.4 | 64.3 | 62.7 | 62.0 |
| 60 | 78.2 | 75.0 | 76.8 | 73.5 | 72.0 | 71.2 |
| 90 | 88.7 | 85.4 | 86.7 | 84.4 | 83.2 | 82.7 |
| 120 | 94.0 | 90.9 | 92.1 | 90.8 | 90.0 | 89.5 |
| 150 | 96.8 | 94.5 | 95.0 | 94.3 | 93.8 | 93.0 |
| 180 | 98.2 | 96.1 | 96.9 | 96.2 | 95.8 | 95.5 |

Table 2:

| Composition A: Release (in-vitro-dissolution) of prilocaine hydrochloride, intermediate precision (n=6), paddle, 75 rpm | | | | | | |
|---|---|---|---|---|---|---|
| Dissolution [%] | | | | | | |
| Withdrawal [min] | Gel A1 | Gel A2 | Gel A3 | Gel A4 | Gel A5 | Gel A6 |
| 5 | 15.2 | 15.0 | 15.9 | 15.0 | 15.0 | 15.0 |
| 10 | 23.7 | 23.6 | 25.6 | 23.0 | 23.7 | 24.2 |
| 20 | 38.5 | 38.0 | 40.8 | 37.4 | 38.7 | 38.9 |
| 30 | 49.7 | 48.5 | 52.5 | 48.3 | 49.4 | 50.4 |
| 45 | 62.2 | 60.7 | 65.1 | 60.5 | 61.8 | 63.2 |
| 60 | 70.6 | 69.3 | 73.7 | 68.8 | 70.6 | 71.5 |
| 90 | 81.8 | 79.5 | 84.0 | 80.0 | 81.6 | 82.3 |
| 120 | 87.9 | 85.3 | 89.3 | 85.7 | 87.8 | 89.0 |
| 150 | 90.6 | 88.8 | 92.3 | 88.9 | 91.1 | 92.8 |
| 180 | 93.6 | 90.7 | 94.0 | 90.9 | 93.4 | 94.4 |

Table 3:

| Composition B: Release (in-vitro-dissolution) of prilocaine hydrochloride, precision (n=6), paddle, 75 rpm | | | | | | |
|---|---|---|---|---|---|---|
| Dissolution [%] | | | | | | |
| Withdrawal [min] | Gel B1 | Gel B2 | Gel B3 | Gel B4 | Gel B5 | Gel B6 |
| 5 | 14.1 | 11.8 | 11.6 | 15.9 | 14.9 | 13.9 |
| 10 | 23.7 | 20.7 | 20.5 | 25.6 | 24.0 | 22.8 |
| 20 | 38.5 | 34.3 | 34.7 | 40.8 | 38.9 | 37.0 |
| 30 | 49.9 | 44.6 | 45.2 | 51.5 | 50.2 | 47.5 |
| 45 | 61.9 | 56.1 | 57.1 | 63.3 | 63.2 | 59.2 |

(continued)

| Composition B: Release (in-vitro-dissolution) of prilocaine hydrochloride, precision (n=6), paddle, 75 rpm | | | | | | |
|---|---|---|---|---|---|---|
| Dissolution [%] | | | | | | |
| Withdrawal [min] | Gel B1 | Gel B2 | Gel B3 | Gel B4 | Gel B5 | Gel B6 |
| 60 | 70.9 | 64.4 | 66.1 | 72.0 | 71.9 | 68.1 |
| 90 | 81.5 | 75.7 | 77.6 | 82.3 | 83.9 | 78.6 |
| 120 | 87.0 | 82.2 | 84.1 | 87.9 | 90.7 | 84.5 |
| 150 | 90.5 | 86.0 | 88.2 | 90.6 | 94.3 | 87.5 |
| 180 | 91.8 | 88.4 | 90.5 | 92.7 | 96.9 | 89.1 |
| 210 | 94.6 | 92.2 | 93.4 | 95.8 | 100.1 | 92.2 |
| 240 | 95.3 | 93.7 | 94.3 | 96.5 | 100.8 | 92.8 |

Table 4:

| Composition B: Release (in-vitro-dissolution) of prilocaine hydrochloride, intermediate precision (n=6), paddle, 75 rpm | | | | | | |
|---|---|---|---|---|---|---|
| Dissolution [%] | | | | | | |
| Withdrawal [min] | Gel B1 | Gel B2 | Gel B3 | Gel B4 | Gel B5 | Gel B6 |
| 5 | 15.6 | 15.2 | 15.5 | 15.1 | 14.9 | 16.0 |
| 10 | 25.1 | 24.0 | 25.3 | 24.0 | 23.8 | 25.8 |
| 20 | 39.7 | 38.2 | 40.1 | 38.2 | 37.8 | 40.1 |
| 30 | 50.6 | 48.7 | 50.9 | 48.4 | 48.3 | 51.0 |
| 45 | 62.1 | 59.7 | 62.6 | 59.6 | 59.5 | 61.8 |
| 60 | 70.6 | 68.6 | 71.4 | 68.1 | 68.4 | 70.3 |
| 90 | 80.7 | 79.3 | 81.8 | 78.3 | 79.2 | 80.2 |
| 120 | 85.8 | 85.0 | 87.2 | 84.2 | 84.9 | 85.6 |
| 150 | 88.5 | 88.5 | 90.0 | 87.2 | 88.1 | 88.3 |
| 180 | 90.6 | 90.1 | 91.6 | 89.2 | 90.0 | 90.0 |
| 210 | 93.0 | 93.3 | 94.1 | 91.8 | 92.8 | 91.7 |
| 240 | 93.3 | 94.2 | 94.8 | 92.8 | 93.5 | 92.8 |

Table 5:

| Composition C (Sample 1): Release (in-vitro-dissolution) of prilocaine hydrochloride, precision (n=6), paddle, 75 rpm | | | | | | |
|---|---|---|---|---|---|---|
| Dissolution [%] | | | | | | |
| Withdrawal [min] | Gel $C_1$1 | Gel $C_1$2 | Gel $C_1$3 | Gel $C_1$4 | Gel $C_1$5 | Gel $C_1$6 |
| 5 | 15.4 | 14.8 | 13.3 | 16.1 | 14.1 | 14.6 |
| 10 | 24.2 | 23.1 | 20.5 | 24.7 | 21.3 | 22.2 |
| 20 | 38.1 | 35.8 | 32.0 | 39.1 | 34.4 | 35.1 |
| 30 | 47.9 | 45.9 | 41.3 | 49.7 | 43.9 | 44.4 |
| 45 | 59.7 | 57.8 | 52.4 | 62.0 | 56.0 | 55.5 |
| 60 | 68.1 | 66.5 | 60.5 | 70.6 | 64.2 | 64.2 |

(continued)

| Composition C (Sample 1): Release (in-vitro-dissolution) of prilocaine hydrochloride, precision (n=6), paddle, 75 rpm | | | | | | |
|---|---|---|---|---|---|---|
| Dissolution [%] | | | | | | |
| Withdrawal [min] | Gel $C_1$1 | Gel $C_1$2 | Gel $C_1$3 | Gel $C_1$4 | Gel $C_1$5 | Gel $C_1$6 |
| 90 | 79.1 | 77.4 | 72.0 | 81.9 | 76.0 | 75.7 |
| 120 | 85.6 | 84.2 | 80.4 | 88.1 | 83.2 | 82.2 |
| 150 | 89.7 | 88.6 | 85.3 | 91.5 | 88.2 | 86.8 |
| 180 | 92.2 | 91.1 | 88.7 | 93.6 | 91.1 | 89.2 |

Table 6:

| Composition C (Sample 1): Release (in-vitro-dissolution) of prilocaine hydrochloride, intermediate precision (n=6), paddle, 75 rpm | | | | | | |
|---|---|---|---|---|---|---|
| Dissolution [%] | | | | | | |
| Withdrawal [min] | Gel $C_1$1 | Gel $C_1$2 | Gel $C_1$3 | Gel $C_1$4 | Gel $C_1$5 | Gel $C_1$6 |
| 5 | 12.0 | 12.8 | 14.1 | 14.9 | 13.1 | 13.2 |
| 10 | 23.6 | 25.9 | 28.5 | 29.2 | 25.8 | 26.7 |
| 20 | 36.3 | 40.1 | 43.6 | 45.8 | 38.2 | 41.8 |
| 30 | 45.6 | 50.8 | 55.1 | 57.4 | 47.2 | 52.4 |
| 45 | 56.6 | 63.0 | 67.8 | 70.6 | 57.8 | 64.3 |
| 60 | 64.6 | 71.7 | 76.9 | 79.6 | 65.6 | 73.3 |
| 90 | 76.6 | 83.5 | 88.0 | 90.9 | 77.4 | 84.8 |
| 120 | 84.2 | 91.3 | 94.7 | 96.5 | 85.5 | 92.2 |
| 150 | 89.9 | 96.0 | 98.0 | 99.8 | 91.1 | 96.7 |
| 180 | 93.9 | 99.0 | 100.4 | 101.5 | 95.1 | 99.3 |

Table 7:

| Composition C (Sample 2): Release (in-vitro-dissolution) of prilocaine hydrochloride, precision (n=6), paddle, 75 rpm | | | | | | |
|---|---|---|---|---|---|---|
| Dissolution [%] | | | | | | |
| Withdrawal [min] | Gel $C_2$1 | Gel $C_2$2 | Gel $C_2$3 | Gel $C_2$4 | Gel $C_2$5 | Gel $C_2$6 |
| 5 | 17.9 | 18.0 | 16.7 | 17.5 | 14.6 | 14.5 |
| 10 | 28.5 | 27.9 | 27.2 | 28.2 | 24.5 | 25.1 |
| 20 | 44.6 | 43.2 | 42.7 | 44.0 | 40.0 | 40.7 |
| 30 | 56.3 | 54.5 | 54.2 | 55.4 | 51.5 | 52.3 |
| 45 | 69.0 | 66.7 | 66.6 | 68.2 | 63.9 | 64.7 |
| 60 | 77.2 | 75.2 | 75.5 | 77.3 | 72.6 | 73.4 |
| 90 | 87.2 | 85.4 | 86.0 | 87.4 | 83.2 | 83.6 |
| 120 | 92.4 | 91.2 | 91.9 | 93.7 | 88.8 | 89.0 |
| 150 | 95.1 | 94.3 | 95.2 | 96.4 | 91.9 | 91.9 |
| 180 | 96.8 | 96.0 | 97.5 | 98.4 | 93.7 | 93.7 |

The results are also shown in Figures 1 to 7, which correspond to tables 1 to 7 respectively.

Comparative Example 5

Xylonest®, available from Astra Zeneca, may be used as comparative composition X. Xylonest® is a solution for injection comprising 5 mg prilocaine hydrochloride per 1 ml solution. Other excipients in Xylonest ® are: water, sodium chloride, sodium hydroxide and hydrochloric acid 7% for pH adjustment. Xylonest®, which does not comprise hyaluronic acid or any derivative thereof, rapidly releases prilocaine hydrochloride.

The similarity between compositions A, B and C according to examples 1-3 and composition X (Xylonest®) in terms of the rapid release of prilocaine hydrochloride can be confirmed by determining the dissolution characteristics of composition X (Xylonest®) according to the method of example 4.

The following sample was tested:

Composition X: Xylonest® 0.5 %

The results of the test are presented in the following tables 8 and 9. The results are also used for validation of the dissolution testing method. Therefore the tables are titled with "precision" (n=6) and "intermediate precision" (n=6). The requirements of the guideline CPMP/EWP/QWP/1401/98: "12 individual values for every time point for each formulation" is fulfilled.

Table 8:

| Composition X: Release (in-vitro-dissolution) of prilocaine hydrochloride, precision (n=6), paddle, 75 rpm | | | | | | |
|---|---|---|---|---|---|---|
| **Dissolution [%]** | | | | | | |
| Withdrawal [min] | Gel X1 | Gel X2 | Gel X3 | Gel X4 | Gel X5 | Gel X6 |
| 5 | 16.7 | 17.0 | 13.5 | 15.8 | 17.4 | 14.3 |
| 10 | 26.8 | 25.3 | 21.9 | 23.4 | 26.5 | 24.0 |
| 20 | 43.3 | 39.5 | 35.9 | 36.4 | 40.8 | 40.0 |
| 30 | 55.4 | 50.9 | 47.1 | 47.0 | 51.9 | 52.5 |
| 45 | 67.4 | 63.2 | 59.2 | 59.0 | 64.2 | 65.8 |
| 60 | 75.8 | 72.2 | 68.5 | 68.1 | 73.5 | 75.2 |
| 90 | 86.4 | 84.0 | 81.1 | 80.7 | 85.2 | 86.8 |
| 120 | 92.5 | 90.9 | 88.5 | 89.1 | 91.6 | 92.9 |
| 150 | 95.9 | 95.2 | 92.9 | 93.7 | 95.2 | 96.1 |
| 180 | 97.6 | 97.1 | 95.9 | 96.5 | 97.6 | 98.1 |

Table 9:

| Composition X: Release (in-vitro-dissolution) of prilocaine hydrochloride, intermediate precision (n=6), paddle, 75 rpm | | | | | | |
|---|---|---|---|---|---|---|
| **Dissolution [%]** | | | | | | |
| Withdrawal [min] | Gel X1 | Gel X2 | Gel X3 | Gel X4 | Gel X5 | Gel X6 |
| 5 | 15,4 | 16,6 | 14,6 | 13,7 | 13,1 | 16,1 |
| 10 | 26,0 | 26,5 | 23,9 | 22,9 | 22,2 | 26,5 |
| 20 | 42,7 | 42,7 | 38,9 | 37,7 | 37,0 | 42,7 |
| 30 | 55,2 | 54,8 | 50,3 | 49,4 | 48,8 | 54,7 |
| 45 | 68,6 | 67,3 | 62,7 | 62,1 | 61,8 | 67,8 |
| 60 | 78,2 | 76,0 | 71,8 | 71,5 | 71,2 | 76,3 |
| 90 | 88,8 | 86,8 | 83,6 | 82,7 | 83,6 | 87,1 |

(continued)

| Composition X: Release (in-vitro-dissolution) of prilocaine hydrochloride, intermediate precision (n=6), paddle, 75 rpm | | | | | | |
|---|---|---|---|---|---|---|
| **Dissolution [%]** | | | | | | |
| Withdrawal [min] | Gel X1 | Gel X2 | Gel X3 | Gel X4 | Gel X5 | Gel X6 |
| 120 | 94,4 | 92,4 | 90,0 | 89,3 | 90,2 | 92,6 |
| 150 | 97,0 | 95,5 | 93,6 | 93,0 | 93,8 | 95,2 |
| 180 | 98,3 | 96,8 | 95,6 | 95,3 | 95,7 | 97,1 |

The results are also shown in Figures 8 and 9, which correspond to tables 8 and 9 respectively.

By means of a similarity calculation it can be shown that the dissolution of prilocaine hydrochloride in the compositions A, B and C according to examples 1-3 is similar to the dissolution of prilocaine hydrochloride in composition X (Xylonest® 0.5%).

## Claims

**1.** A composition of matter comprising

(a1) hyaluronic acid and/or a pharmaceutically acceptable hyaluronate salt,
(a2) cross-linked hyaluronic acid and/or a pharmaceutically acceptable cross-linked hyaluronate salt, having a cross-linking degree of 35 to 65%,
(b) prilocaine and/or a pharmaceutically acceptable salt thereof, and optionally
(c) at least one additive.

**2.** A composition of matter as claimed in claim 1, wherein the composition has a dynamic viscosity (20°C, 10 revolutions per second) of 5,000 to 20,000 mPa*s, preferably 6,000 to 18,000 mPa*s, more preferably 8,000 to 16,000 mPa*s.

**3.** A composition of matter as claimed in claim 1 or 2, wherein component (a1) is contained in an amount of 0.001 to 5% by weight, preferably 0.01 to 2% by weight, more preferably 0.01 to 0.5 % by weight based on the total weight of the composition.

**4.** A composition of matter as claimed in any one of claims 1-3, wherein component (a1) has an average molecular weight of 2,200,000 to 3,800,000 Dalton, preferably 2,400,000 to 3,600,000, more preferably 2,500,000 to 3,500,000 Dalton, most preferably 2,900,000 to 3,500,000 Dalton.

**5.** A composition of matter as claimed in any one of claims 1-4, wherein the hyaluronate salt according to component (a1) is selected from the group consisting of sodium hyaluronate, potassium hyaluronate, calcium hyaluronate and any mixture thereof.

**6.** A composition of matter as claimed in any one of claims 1-5, wherein the hyaluronate salt according to component (a1) is sodium hyaluronate.

**7.** A composition of matter as claimed in any one of claims 1-6, wherein component (b) is contained in an amount of 0.001 to 5% by weight, preferably 0.01 to 3% by weight, more preferably 0.1 to 1% by weight based on the total weight of the composition.

**8.** A composition of matter as claimed in any one of claims 1-7, wherein component (b) is prilocaine hydrochloride.

**9.** A composition of matter as claimed in any one of claims 1-8, wherein the composition comprises as component (c) one or more additives, selected from the group consisting of buffering agents, osmolality adjusters, pH adjusters, thickening agents, gelling agents, preservatives, stabilizers, solubilizers, emulsifying agents, anti-oxidants, electrolytes, radical scavengers, vitamins, enzymes, scents, coloring agents, pigments, melanin, light protection filters, waxes, resins, oils, esters, alcohols, and polyols.

**10.** A composition of matter as claimed in any one of claims 1-9, wherein the composition comprises as component (c) at least a buffering agent selected from the group consisting of disodium hydrogen phosphate dihydrate, sodium dihydrogen phosphate dihydrate and any mixture thereof, and optionally sodium chloride as an osmolality adjuster.

**11.** A composition of matter as claimed in any one of claims 1-10, wherein component (a2) is contained in an amount of 0.01 to 10% by weight, preferably 0.1 to 3% by weight, more preferably 0.5 to 2.5% by weight based on the total weight of the composition.

**12.** A composition of matter as claimed in any one of claims 1-11, wherein component (a2) has a molecular weight of 500,000 to 2,000,000, preferably 600,000 to 1,800,000, more preferably 700,000 to 1,500,000 Dalton, most preferably 900,000 to 1,100,000 Dalton.

**13.** A composition of matter as claimed in any one of claims 1-12, wherein the cross-linked hyaluronate salt according to component (a2) is selected from the group consisting of cross-linked sodium hyaluronate, cross-linked potassium hyaluronate, cross-linked calcium hyaluronate, and any mixture thereof, which was preferably cross-linked with a diglycidyl ether, more preferably with 1,4-butanediol diglycidyl ether (BDDE), as cross-linking agent.

**14.** A composition of matter as claimed in any one of claims 1-13, wherein component (a2) has a cross-linking degree of preferably 40 to 60%, more preferably 42 to 55 %.

**15.** A composition of matter as claimed in any one of claims 1-14, wherein component (a2) has a particle size distribution **characterized in that** at least 95 vol.-% of the particles have a diameter lower than 300 μm, at least 90 vol.-% of the particles have a diameter lower than 250 μm, at least 50 vol.-% of the particles have a diameter lower than 120 μm, at least 10 vol.-% of the particles have a diameter lower than 50 μm, at least 5 vol.-% of the particles have a diameter lower than 45 μm, wherein the vol.-% are based on the total volume of component (a2) in the composition.

**16.** A composition of matter as claimed in any one of claims 1-15, wherein component (a2) is obtainable by

(1) cross-linking hyaluronic acid and/or hyaluronate in solution to a cross-linking degree of 35 to 65%,
(2) filtering and washing the cross-linked hyaluronic acid and/or cross-linked hyaluronate obtained according to step (1),
(3) increasing the surface area of the cross-linked hyaluronic acid and/or cross-linked hyaluronate obtained according to step (2),
(4) further cross-linking the cross-linked hyaluronic acid and/or cross-linked hyaluronate obtained according to step (3) in the presence of hyaluronic acid and/or hyaluronate, and
(5) washing the cross-linked hyaluronic acid and/or cross-linked hyaluronate obtained according to step (4).

**17.** A composition of matter as claimed in claim 16, wherein the cross-linked hyaluronic acid and/or cross-linked hyaluronate obtained according to step (1) is cross-linked via ether- and/or ester- and/or sulfon- and/or amine- and/or imine- and/or amide-bonds, preferably via formation of ether- and/or ester- and/or sulfon-bonds, more preferably via ether- or sulfon-bonds in the presence of a cross-linking agent selected from the group consisting of formaldehyde, glutaraldehyde, divinyl sulfone, polyanhydrides, polyaldehydes, polyhydric alcohols, carbodiimides, carboxylic acid chlorides, sulfonic acid chlorides, epichlorohydrin, ethylene glycol, diglycidyl ethers such as butanediol diglycidyl ether, polyethylene glycol diglycidyl ether, and polypropylene gyclol diglycidyl ether, polyglycerol polyglycidyl ethers, and bis- or polyepoxides, preferably in the presence of butanediol diglycidyl ether or divinyl sulfone.

**18.** A composition of matter as claimed in claim 16 or 17, wherein the cross-linked hyaluronic acid and/or cross-linked hyaluronate obtained according to step (4) is cross-linked via ether- and/or ester- and/or sulfon- and/or amine- and/or imine- and/or amide-bonds, preferably via formation of ether- and/or ester- and/or sulfon-bonds, more preferably via ether- or sulfon-bonds in the presence of a cross-linking agent selected from the group consisting of formaldehyde, glutaraldehyde, divinyl sulfone, polyanhydrides, polyaldehydes, polyhydric alcohols, carbodiimides, carboxylic acid chlorides, sulfonic acid chlorides, epichlorohydrin, ethylene glycol, diglycidyl ethers such as butanediol diglycidyl ether, polyethylene glycol diglycidyl ether, and polypropylene gyclol diglycidyl ether, polyglycerol polyglycidyl ethers, and bis- or polyepoxides, preferably in the presence of butanediol diglycidyl ether or divinyl sulfone.

**19.** A composition of matter as claimed in any one of claims 1-18, obtainable by

(i) dissolving component (b) in an aqueous solution comprising as component (c1) at least one additive,

(ii) dispersing component (a1) in an aqueous solution comprising as component (c2) at least one additive,
(iii) mixing component (a2), the solution of component (b) according to step (i) and the dispersion of component (a1) according to step (ii) with an aqueous solution comprising as component (c3) at least one additive,
wherein the additives according to components (c1), (c2) and (c3) may be identical or different.

**20.** The composition of matter as claimed in any one of claims 1 to 19 for use

(I) in the treatment of skin and/or mucous membrane changes,
(II) in cosmetic surgery, and/or
(III) in ophthalmologic surgery

**21.** The composition of matter as claimed in claim 20, wherein the composition of matter is administered by injection.

**22.** The composition of matter as claimed in claim 21 for use in the simultaneous treatment and/or prevention of pain and/or acute inflammation caused by the injection.

**23.** The composition of matter as claimed in any one of claims 1 to 19 for use

(IV) in the treatment of degenerative joint diseases including osteoarthritis and/or traumatic joint diseases,
(V) in the treatment of articular cartilage and/or cartilage bone defects,
(VI) in the treatment of meniscus lesions and/or intervertebral disc lesions,
(VII) in the treatment of arthrosis and/or articular rheumatism, and/or
(VIII) in the treatment of osteochondritis dissecans and/or flake fractures.

**24.** The composition of matter as claimed in any one of claims 1 to 19 for use

(IX) in the treatment of osteoarthritis,
(X) in the treatment and/or prevention of acute inflammation in a subject suffering from osteoarthritis,
(XI) in the treatment of osteoarthritis and simultaneous treatment and/or prevention of acute inflammation,
(XII) in the treatment and/or prevention of pain in a subject suffering from osteoarthritis, and/or
(XIII) in the treatment of osteoarthritis and simultaneous treatment and/or prevention of pain.

**25.** The composition of matter as claimed in claim 23 or 24, wherein the composition of matter is administered by intra-articular injection.

**26.** The composition of matter as claimed in claim 25 for use in the simultaneous treatment and/or prevention of pain and/or acute inflammation caused by the intra-articular injection.

**27.** The composition of matter as claimed in any one of claims claim 1-26, wherein component (b) is released in an amount of at least 20% after 10 minutes, at least 50% after 45 minutes, and at least 80% after 120 minutes, wherein the % value is based on the total amount of component (b) in the composition.

## Patentansprüche

**1.** Eine Stoffzusammensetzung umfassend

(a1) Hyaluronsäure und/oder ein pharmazeutisch verträgliches Hyaluronatsalz,
(a2) vernetzte Hyaluronsäure und/oder ein pharmazeutisch verträgliches vernetztes Hyaluronatsalz, das einen Vernetzungsgrad von 35 bis 65% aufweist,
(b) Prilocain und/oder eines seiner pharmazeutisch verträglichen Salze und gegebenenfalls
(c) wenigstens einen Zusatzstoff.

**2.** Eine Stoffzusammensetzung gemäß Anspruch 1, wobei die Zusammensetzung eine dynamische Viskosität (20°C, 10 Umdrehungen pro Sekunde) von 5 000 bis 20 000 mPa*s, bevorzugt 6 000 bis 18 000 mPa*s, besonders bevorzugt 8 000 bis 16 000 mPa*s aufweist.

**3.** Eine Stoffzusammensetzung gemäß Anspruch 1 oder 2, wobei die Komponente (a1) in einer Menge von 0,001 bis

5 Gew.-%, bevorzugt 0,01 bis 2 Gew.-%, besonders bevorzugt 0,01 bis 0,5 Gew.-%, bezogen auf das Gesamtgewicht der Stoffzusammensetzung, enthalten ist.

**4.** Eine Stoffzusammensetzung gemäß einem der Ansprüche 1-3, wobei die Komponente (a1) ein mittleres Molekulargewicht von 2 200 000 bis 3 800 000 Dalton, bevorzugt 2 400 000 bis 3 600 000, besonders bevorzugt 2 500 000 bis 3 500 000 Dalton, ganz besonders bevorzugt 2 900 000 bis 3 500 000 Dalton, aufweist.

**5.** Eine Stoffzusammensetzung gemäß einem der Ansprüche 1-4, wobei das Hyaluronatsalz gemäß der Komponente (a1) ausgewählt wird aus der Gruppe bestehend aus Natriumhyaluronat, Kaliumhyaluronat, Calciumhyaluronat und deren Mischungen.

**6.** Eine Stoffzusammensetzung gemäß einem der Ansprüche 1-5, wobei das Hyaluronatsalz gemäß der Komponente (a1) ein Natriumhyaluronat ist.

**7.** Eine Stoffzusammensetzung gemäß einem der Ansprüche 1-6, wobei die Komponente (b) in einer Menge von 0,001 bis 5 Gew.-%, bevorzugt 0,01 bis 3 Gew.-%, besonders bevorzugt 0,1 bis 1 Gew.-%, basierend auf dem Gesamtgewicht der Stoffzusammensetzung, enthalten ist.

**8.** Eine Stoffzusammensetzung gemäß einem der Ansprüche 1-7, wobei die Komponente (b) Prilocainhydrochlorid ist.

**9.** Eine Stoffzusammensetzung gemäß einem der Ansprüche 1-8, wobei die Stoffzusammensetzung als Komponente (c) ein oder mehrere Zusatzstoffe ausgewählt aus der Gruppe bestehend aus Puffermitteln, Mitteln zur Regulierung der Osmolalität, Mitteln zur Regulierung des pH-Wertes, Verdickungsmitteln, Geliermitteln, Konservierungsmitteln, Stabilisatoren, Lösungsvermittlern, Emulgatoren, Antioxidationsmitteln, Elektrolyten, Radikalfängern, Vitaminen, Enzymen, Düften, Farbstoffen, Pigmenten, Melanin, Lichtschutzfiltern, Wachsen, Harzen, Ölen, Estern, Alkoholen und Polyolen enthält.

**10.** Eine Stoffzusammensetzung gemäß einem der Ansprüche 1-9, wobei die Stoffzusammensetzung als Komponente (c) wenigstens ein Puffermittel ausgewählt aus der Gruppe bestehend aus inatriumhydrogenphosphatdihydrat, Natriumdihydrogenphosphatdihydrat und deren Mischungen und gegebenenfalls Natriumchlorid als Mittel zur Regulierung der Osmolalität umfasst.

**11.** Eine Stoffzusammensetzung gemäß einem der Ansprüche 1-10, wobei die Komponente (a2) in einer Menge von 0,01 bis 10 Gew.-%, bevorzugt 0,1 bis 3 Gew.-%, besonders bevorzugt 0,5 bis 2,5 Gew.-%, basierend auf dem Gesamtgewicht der Zusammensetzung, enthalten ist.

**12.** Eine Stoffzusammensetzung gemäß einem der Ansprüche 1-11, wobei die Komponente (a2) ein Molekulargewicht von 500 000 bis 2 000 000, bevorzugt 600 000 bis 1 800 000, besonders bevorzugt 700 000 bis 1 500 000 Dalton, ganz besonders bevorzugt 900 000 bis 1 100 000 Dalton, aufweist.

**13.** Eine Stoffzusammensetzung gemäß einem der Ansprüche 1-12, wobei das vernetzte Hyaluronatsalz gemäß der Komponente (a2) ausgewählt wird aus der Gruppe bestehend aus vernetztem Natriumhyaluronat, vernetztem Kaliumhyaluronat, vernetztem Calciumhyaluronat und deren Mischungen, die bevorzugt mit einem Diglycidylether, besonders bevorzugt mit 1,4-Butandioldiglycidylether (BDDE), als Vernetzungsmittel vernetzt wurden.

**14.** Eine Stoffzusammensetzung gemäß einem der Ansprüche 1-13, wobei die Komponente (a2) einen Vernetzungsgrad von bevorzugt 40 bis 60%, besonders bevorzugt 42 bis 55% aufweist.

**15.** Eine Stoffzusammensetzung gemäß einem der Ansprüche 1-14, wobei die Komponente (a2) eine Partikelgrößenverteilung aufweist, die **dadurch gekennzeichnet ist, dass** wenigstens 95 Vol.-% der Partikel einen Durchmesser kleiner als 300 μm, wenigstens 90 Vol.-% der Partikel einen Durchmesser kleiner als 250 μm, wenigstens 50 Vol.-% der Partikel einen Durchmesser kleiner als 120 μm, wenigstens 10 Vol.-% der Partikel einen Durchmesser kleiner als 50 μm, wenigstens 5 Vol.-% der Partikel einen Durchmesser kleiner als 45 μm aufweisen, wobei die Vol.-%-Angaben auf das Gesamtvolumen der Komponente (a2) in der Zusammensetzung bezogen sind.

**16.** Eine Stoffzusammensetzung gemäß einem der Ansprüche 1-15, wobei die Komponente (a2) erhältlich ist durch

(1) Vernetzen von Hyaluronsäure und/oder Hyaluronat in Lösung, um einen Vernetzungsgrad von 35 bis 65%

zu erhalten,

(2) Filtern und Waschen der vernetzten Hyaluronsäure und/oder des vernetzten Hyaluronats, die bzw. das gemäß Schritt (1) erhalten wurde,

(3) Erhöhen des Oberflächenbereichs der vernetzten Hyaluronsäure und/oder des vernetzten Hyaluronats, die bzw. das gemäß Schritt (2) erhalten wurde,

(4) weiteres Vernetzen der vernetzten Hyaluronsäure und/oder des vernetzten Hyaluronats, die bzw. das gemäß Schritt (3) erhalten wurde, in der Anwesenheit von Hyaluronsäure und/oder Hyaluronat, und

(5) Waschen der vernetzten Hyaluronsäure und/oder des vernetzten Hyaluronats, die bzw. das gemäß Schritt (4) erhalten wurde.

**17.** Eine Stoffzusammensetzung gemäß Anspruch16, wobei die vernetzte Hyaluronsäure und/oder das vernetzte Hyaluronat, die bzw. das gemäß Schritt (1) erhalten wurde, über Ether- und/oder Ester- und/oder Sulfon- und/oder Amin- und/oder Imin- und/oder Amid-Bindungen vernetzt ist, bevorzugt durch Bildung von Ether- und/oder Ester- und/oder Sulfon-Bindungen vernetzt ist, besonders bevorzugt durch Ether- oder Sulfon-Bindungen in der Anwesenheit eines Vernetzungsmittels ausgewählt aus der Gruppe bestehend aus Formaldehyd, Glutaraldehyd, Divinylsulfon, Polyanhydriden, Polyaldehyden, polyhydrischen Alkoholen, Carbodiimiden, Carbonsäurechloriden, Sulfonsäurechloriden, Epichlorhydrin, Ethylenglykol, Diglycidylethern, wie beispielsweise Butandioldiglycidylether, Polyethylenglykoldiglycidylether und Polypropylengycloldiglycidylether, Polyglycerolpolyglycidylethern und bis- oder Polyepoxide, bevorzugt in der Anwesenheit von Butandioldiglycidylether oder Divinylsulfon, vernetzt wurde.

**18.** Eine Stoffzusammensetzung gemäß Anspruch16 oder 17, wobei die vernetzte Hyaluronsäure und/oder das vernetzte Hyaluronat, die bzw. das gemäß Schritt (4) erhalten wurde, über Ether- und/oder Ester- und/oder Sulfon- und/oder Amin- und/oder Imin- und/oder Amid-Bindungen vernetzt ist, bevorzugt durch Bildung von Ether- und/oder Ester- und/oder Sulfon-Bindungen vernetzt ist, besonders bevorzugt durch Ether- oder Sulfon-Bindungen in der Anwesenheit eines Vernetzungsmittels ausgewählt aus der Gruppe bestehend aus Formaldehyd, Glutaraldehyd, Divinylsulfon, Polyanhydriden, Polyaldehyden, polyhydrischen Alkoholen, Carbodiimiden, Carbonsäurechloriden, Sulfonsäurechloriden, Epichlorhydrin, Ethylenglykol, Diglycidylethern, wie beispielsweise Butandioldiglycidylether, Polyethylenglykoldiglycidylether und Polypropylengycloldiglycidylether, Polyglycerolpolyglycidylethern und bis- oder Polyepoxiden, bevorzugt in der Anwesenheit von Butandioldiglycidylether oder Divinylsulfon vernetzt wurde.

**19.** Eine Stoffzusammensetzung gemäß einem der Ansprüche 1-18, erhältlich durch

(i) Lösen der Komponente (b) in einer wässrigen Lösung umfassend als Komponente (c1) wenigstens einen Zusatzstoff,

(ii) Dispergieren der Komponente (a1) in einer wässrigen Lösung umfassend als Komponente (c2) wenigstens einen Zusatzstoff, und

(iii) Mischen der Komponente (a2), der Lösung der Komponente (b) gemäß Schritt (i) und der Dispersion der Komponente (a1) gemäß dem Schritt (ii) mit einer wässrigen Lösung umfassend als Komponente (c3) wenigstens einen Zusatzstoff,

wobei die Zusatzstoffe gemäß den Komponenten (c1), (c2) und (c3) identisch oder verschieden sein können.

**20.** Die Stoffzusammensetzung gemäß einem der Ansprüche 1 bis 19 zur Verwendung

(I) in der Behandlung von Haut- und/oder Schleimhautveränderungen,

(II) in der kosmetischen Chirurgie und/oder

(III) in der ophthalmologischen Chirurgie

**21.** Die Stoffzusammensetzung gemäß Anspruch 20, wobei die Stoffzusammensetzung durch Injektion verabreicht wird.

**22.** Die Stoffzusammensetzung gemäß Anspruch 21 zur Verwendung in der gleichzeitigen Behandlung und/oder Prävention von Schmerzen und/oder akuter Entzündung, die durch die Injektion verursacht werden.

**23.** Die Stoffzusammensetzung gemäß einem der Ansprüche 1 bis 19 zur Verwendung

(IV) in der Behandlung von degenerativen Gelenkerkrankungen einschließlich Osteoarthritis und/oder traumatischen Gelenkerkrankungen,

(V) in der Behandlung von Gelenkknorpel- und/oder Knorpelknochendefekten,

(VI) in der Behandlung von Meniskusläsionen und/oder Bandscheibenläsionen,

(VII) in der Behandlung von Arthrose und/oder Gelenkrheumatismus und/oder
(VIII) in der Behandlung von *Osteochondritis dissecans* und/oder Splitterbrüchen.

**24.** Die Stoffzusammensetzung gemäß einem der Ansprüche 1 bis 19 zur Verwendung

(IX) in der Behandlung von Osteoarthritis,
(X) in der Behandlung und/oder Prävention von akuten Entzündungen in einem Subjekt, das unter Osteoarthritis leidet,
(XI) in der Behandlung von Osteoarthritis und gleichzeitiger Behandlung und/oder Prävention von akuten Entzündungen,
(XII) in der Behandlung und/oder Prävention von Schmerz in einem Subjekt, das unter Osteoarthritis leidet, und/oder
(XIII) in der Behandlung von Osteoarthritis und gleichzeitiger Behandlung und/oder Prävention von Schmerz.

**25.** Die Stoffzusammensetzung gemäß Anspruch 23 oder 24, wobei die Stoffzusammensetzung durch intraartikuläre Injektion verabreicht wird.

**26.** Die Stoffzusammensetzung gemäß Anspruch 25 zur Verwendung in der gleichzeitigen Behandlung und/oder Prävention von Schmerz und/oder akuter Entzündung, die durch die intraartikuläre Injektion verursacht werden.

**27.** Die Stoffzusammensetzung gemäß einem der Ansprüche 1-26, wobei die Komponente (b) in einer Menge von wenigstens 20% nach 10 Minuten, wenigstens 50% nach 45 Minuten und wenigstens 80% nach 120 Minuten freigesetzt wird, wobei die Prozentangaben auf die Gesamtmenge der Komponente (b) in der Zusammensetzung bezogen sind.

## Revendications

**1.** Composition de matière, comprenant

(a1) de l'acide hyaluronique et/ou un sel d'hyaluronate pharmaceutiquement acceptable,
(a2) de l'acide hyaluronique réticulé et/ou un sel d'hyaluronate réticulé pharmaceutiquement acceptable, ayant un degré de réticulation allant de 35 à 65%,
(b) de la prilocaïne ou un sel pharmaceutiquement acceptable de celle-ci, et éventuellement
(c) au moins un additif.

**2.** Composition de matière selon la revendication 1, où la composition possède une viscosité dynamique (20°C, 10 révolutions par seconde) allant de 5000 à 20 000 mPa.s, préférablement de 6000 à 18 000 mPa.s, plus préférablement de 8000 à 16 000 mPa.s.

**3.** Composition de matière selon la revendication 1 ou 2, où le composant (a1) est contenu selon une quantité allant de 0,001 à 5% en poids, préférablement de 0,01 à 2% en poids, plus préférablement de 0,01 à 0,5% en poids, sur la base du poids total de la composition.

**4.** Composition de matière selon l'une quelconque des revendications 1-3, où le composant (a1) possède un poids moléculaire moyen allant de 2 200 000 à 3 800 000 Daltons, préférablement de 2 400 000 à 3 600 000 Daltons, plus préférablement de 2 500 000 à 3 500 000 Daltons, tout préférablement de 2 900 000 à 3 500 000 Daltons.

**5.** Composition de matière selon l'une quelconque des revendications 1-4, où le sel d'hyaluronate selon le composant (a1) est choisi dans le groupe constitué par l'hyaluronate de sodium, l'hyaluronate de potassium, l'hyaluronate de calcium, et un mélange quelconque de ceux-ci.

**6.** Composition de matière selon l'une quelconque des revendications 1-5, où le sel d'hyaluronate selon le composant (a1) est l'hyaluronate de sodium.

**7.** Composition de matière selon l'une quelconque des revendications 1-6, où le composant (b) est contenu selon une quantité allant de 0,001 à 5% en poids, préférablement de 0,01 à 3% en poids, plus préférablement de 0,1 à 1% en poids, sur la base du poids total de la composition.

**8.** Composition de matière selon l'une quelconque des revendications 1-7, où le composant (b) est le chlorhydrate de prilocaïne.

**9.** Composition de matière selon l'une quelconque des revendications 1-8, où la composition comprend, comme composant (c), un ou plusieurs additifs, choisis dans le groupe constitué par les agents tampon, les agents d'ajustement d'osmolalité, les agents d'ajustement du pH, les agents épaississants, les agents gélifiants, les conservateurs, les stabilisants, les solubilisants, les agents émulsifiants, les antioxydants, les électrolytes, les agents anti-radicalaires, les vitamines, les enzymes, les parfums, les agents colorants, les pigments, la mélanine, les filtres photo-protecteurs, les cires, les résines, les huiles, les esters, les alcools et les polyols.

**10.** Composition de matière selon l'une quelconque des revendications 1-9, où la composition comprend comme composant (c) au moins un agent tampon choisi dans le groupe constitué par l'hydrogénophosphate disodique dihydraté, l'hydrogénophosphate de sodium dihydraté, et un mélange quelconque de ceux-ci, et éventuellement du chlorure de sodium comme agent d'ajustement d'osmolalité.

**11.** Composition de matière selon l'une quelconque des revendications 1-10, où le composant (a2) est contenu selon une quantité allant de 0,01 à 10% en poids, préférablement de 0,1 à 3% en poids, plus préférablement de 0,5 à 2,5% en poids, sur la base du poids total de la composition.

**12.** Composition de matière selon l'une quelconque des revendications 1-11, où le composant (a2) possède un poids moléculaire allant de 500 000 à 2 000 000, préférablement de 600 000 à 1 800 000, plus préférablement de 700 000 à 1 500 000 Daltons, tout préférablement de 900 000 à 1 100 000 Daltons.

**13.** Composition de matière selon l'une quelconque des revendications 1-12, où le sel d'hyaluronate réticulé selon le composant (a2) est choisi dans le groupe constitué par l'hyaluronate de sodium réticulé, l'hyaluronate de potassium réticulé, l'hyaluronate de calcium réticulé, et un mélange quelconque de ceux-ci, qui a été réticulé de préférence avec un éther diglycidylique, plus préférablement avec de l'éther diglycidylique de 1,4-butanediol (BDDE) comme agent de réticulation.

**14.** Composition de matière selon l'une quelconque des revendications 1-13, où le composant (a2) possède un degré de réticulation allant préférablement de 40 à 60%, plus préférablement de 42 à 55%.

**15.** Composition de matière selon l'une quelconque des revendications 1-14, où le composant (a2) possède une distribution granulométrique **caractérisée en ce qu'**au moins 95% en volume des particules possèdent un diamètre inférieur à 300 μm, au moins 90% en volume des particules possèdent un diamètre inférieur à 250 μm, au moins 50% en volume des particules possèdent un diamètre inférieur à 120 μm, au moins 10% en volume des particules possèdent un diamètre inférieur à 50 μm, au moins 5% en volume des particules possèdent un diamètre inférieur à 45 μm, où les % en volume sont basés sur le volume total du composant (a2) dans la composition.

**16.** Composition de matière selon l'une quelconque des revendications 1-15, où le composant (a2) peut être obtenu par

(1) réticulation d'acide hyaluronique et/ou d'hyaluronate en solution jusqu'à un degré de réticulation allant de 35 à 65% ;
(2) filtration et lavage de l'acide hyaluronique réticulé et/ou de l'hyaluronate réticulé obtenu selon l'étape (1) ;
(3) augmentation de la surface de l' acide hyaluronique réticulé et/ou de l'hyaluronate réticulé obtenu selon l'étape (2) ;
(4) réticulation supplémentaire de l'acide hyaluronique réticulé et/ou de l'hyaluronate réticulé obtenu selon l'étape (3) en présence d'acide hyaluronique et/ou d'hyaluronate ; et
(5) lavage de l'acide hyaluronique réticulé et/ou de l'hyaluronate réticulé obtenu selon l'étape (4).

**17.** Composition de matière selon la revendication 16, où l'acide hyaluronique réticulé et/ou l'hyaluronate réticulé obtenu selon l'étape (1) est réticulé via des liaisons éther et/ou ester et/ou sulfone et/ou amine et/ou imine et/ou amide, préférablement via la formation de liaisons éther et/ou ester et/ou sulfone, plus préférablement via des liaisons éther ou sulfone en présence d'un agent de réticulation choisi dans le groupe constitué par le formaldéhyde, le glutaraldéhyde, la divinylsulfone, les polyanhydrides, les polyaldéhydes, les alcools polyhydriques, les carbodiimides, les chlorures d'acide carboxylique, les chlorures d'acide sulfonique, l'épichlorhydrine, l'éthylène glycol, les éthers diglycidyliques tels que l'éther diglycidylique de butanediol, l'éther diglycidylique de polyéthylène glycol, et l'éther diglycidylique de polypropylène glycol, les éthers polyglycidyliques de polyglycérol, et les bis- ou polyépoxydes,

préférablement en présence d'éther diglycidylique de butanediol ou de divinylsulfone.

**18.** Composition de matière selon la revendication 16 ou 17, où l'acide hyaluronique réticulé et/ou l'hyaluronate réticulé obtenu selon l'étape (4) est réticulé via des liaisons éther et/ou ester et/ou sulfone et/ou amine et/ou imine et/ou amide, préférablement via la formation de liaisons éther et/ou ester et/ou sulfone, plus préférablement via des liaisons éther ou sulfone en présence d'un agent de réticulation choisi dans le groupe constitué par le formaldéhyde, le glutaraldéhyde, la divinylsulfone, les polyanhydrides, les polyaldéhydes, les alcools polyhydriques, les carbodiimides, les chlorures d'acide carboxylique, les chlorures d'acide sulfonique, l'épichlorhydrine, l'éthylène glycol, les éthers diglycidyliques tels que l'éther diglycidylique de butanediol, l'éther diglycidylique de polyéthylène glycol, et l'éther diglycidylique de polypropylène glycol, les éthers polyglycidyliques de polyglycérol, et les bis- ou polyépoxydes, préférablement en présence d'éther diglycidylique de butanediol ou de divinylsulfone.

**19.** Composition de matière selon l'une quelconque des revendications 1-18, pouvant être obtenue par

(i) solubilisation du composant (b) dans une solution aqueuse comprenant, comme composant (c1), au moins un additif ;
(ii) dispersion du composant (a1) dans une solution aqueuse comprenant, comme composant (c2), au moins un additif ;
(iii) mélange du composant (a2), de la solution de composant (b) selon l'étape (i) et de la dispersion du composant (a1) selon l'étape (ii) avec une solution aqueuse comprenant, comme composant (c3), au moins un additif ;
où les additifs selon les composants (c1), (c2) et (c3) peuvent être identiques ou différents.

**20.** Composition de matière selon l'une quelconque des revendications 1 à 19, pour une utilisation

(I) dans le traitement de changements de la peau et/ou des muqueuses ;
(II) en chirurgie cosmétique ; et/ou
(III) en chirurgie ophtalmologique.

**21.** Composition de matière selon la revendication 20, où la composition de matière est administrée par injection.

**22.** Composition de matière selon la revendication 21, pour une utilisation dans le traitement et/ou la prévention simultanés d'une douleur et/ou d'une inflammation aiguë provoquée par l'injection.

**23.** Composition de matière selon l'une quelconque des revendications 1 à 19, pour une utilisation

(IV) dans le traitement de maladies articulaires dégénératives, y compris l'ostéo-arthrite et/ou les maladies articulaires traumatiques ;
(V) dans le traitement de défauts de cartilage osseux et/ou de cartilage articulaire ;
(VI) dans le traitement de lésions du ménisque et/ou de lésions des disques intervertébraux ;
(VII) dans le traitement de l'arthrose et/ou du rhumatisme articulaire ; et/ou
(VIII) dans le traitement de l'ostéochondrite disséquante et/ou des fractures en écaille.

**24.** Composition de matière selon l'une quelconque des revendications 1 à 19, pour une utilisation

(IX) dans le traitement de l'ostéo-arthrite ;
(X) dans le traitement et/ou la prévention d'inflammations aiguës chez un sujet souffrant d'ostéo-arthrite ;
(XI) dans le traitement de l'ostéo-arthrite et le traitement et/ou la prévention simultanés d'inflammations aiguës ;
(XII) dans le traitement et/ou la prévention de douleurs chez un sujet souffrant d'ostéo-arthrite ; et/ou
(XIII) dans le traitement de l'ostéo-arthrite et le traitement et/ou la prévention simultanés de douleurs.

**25.** Composition de matière selon la revendication 23 ou 24, où la composition de matière est administrée par injection intra-articulaire.

**26.** Composition de matière selon la revendication 25, pour une utilisation dans le traitement et/ou la prévention simultanés d'une douleur et/ou d'une inflammation aiguë provoquée par l'injection intra-articulaire.

**27.** Composition de matière selon l'une quelconque des revendications 1-26, où le composant (b) est libéré selon une quantité d'au moins 20% après 10 minutes, au moins 50% après 45 minutes, et au moins 80% après 120 minutes,

où la valeur en % est basée sur la quantité totale de composant (b) dans la composition.

Figure 1: Composition A: Release (in-vitro-dissolution) of prilocaine hydrochloride, precision (n=6), paddle, 75 rpm

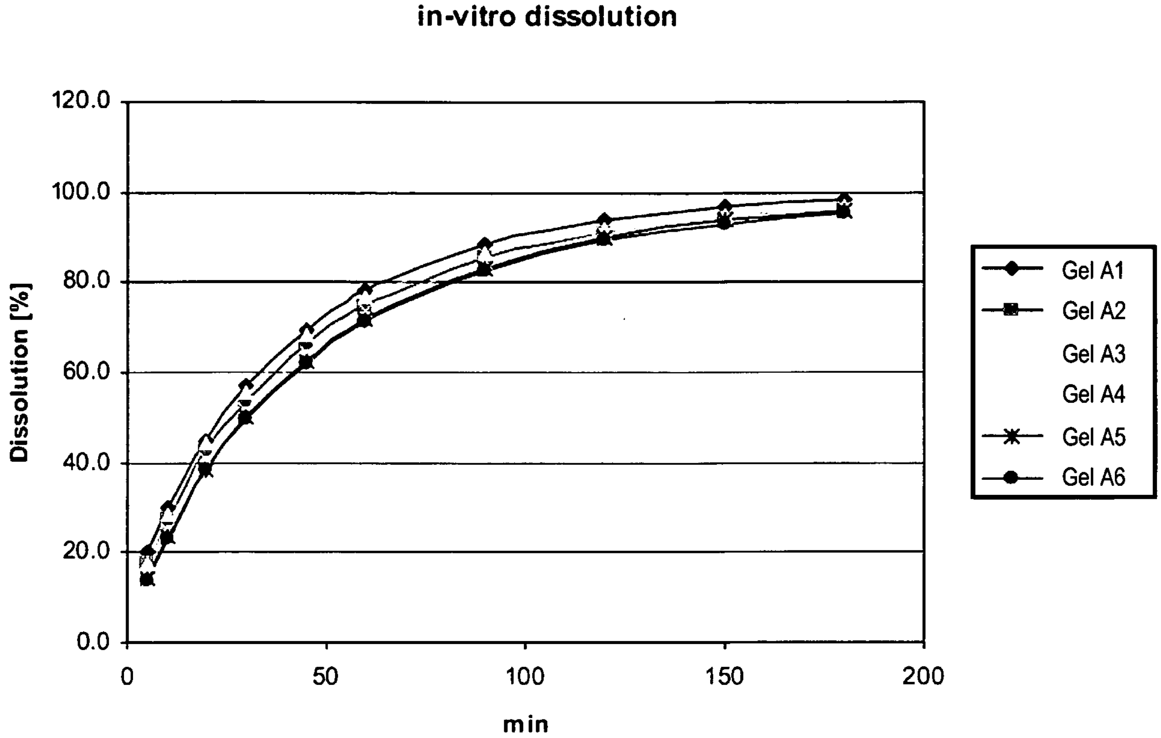


Figure 2: Composition A: Release (in-vitro-dissolution) of prilocaine hydrochloride, intermediate precision (n=6), paddle, 75 rpm

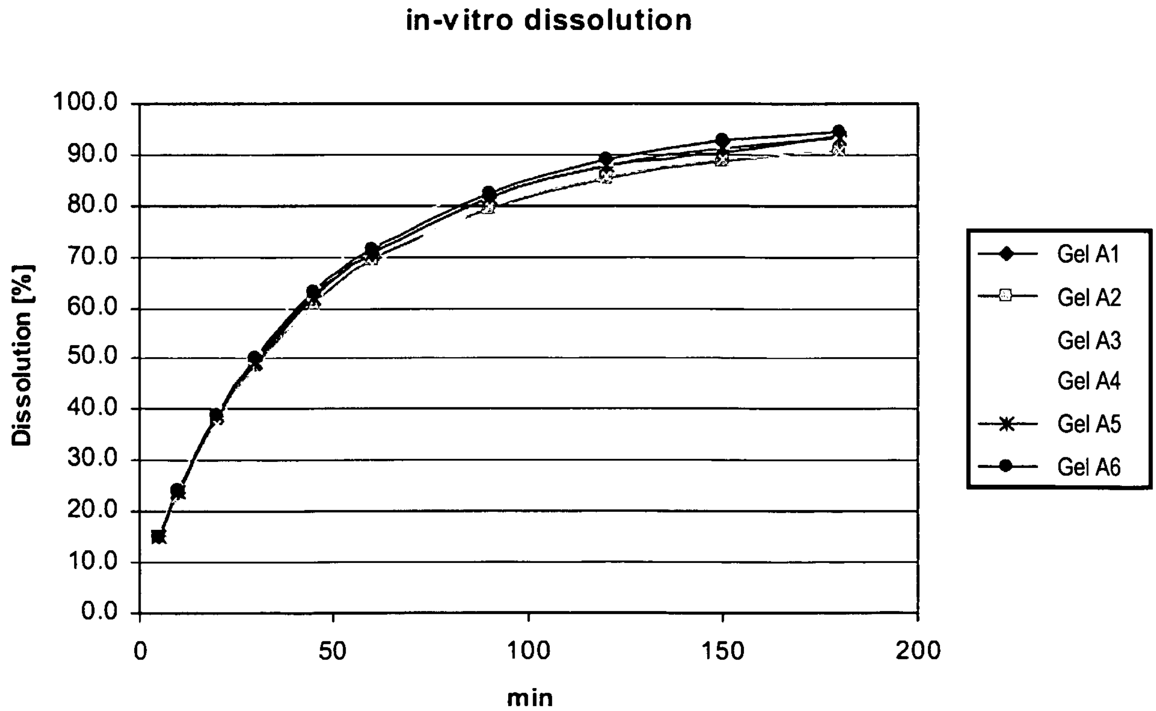

Figure 3: Composition B: Release (in-vitro-dissolution) of prilocaine hydrochloride, precision (n=6), paddle, 75 rpm

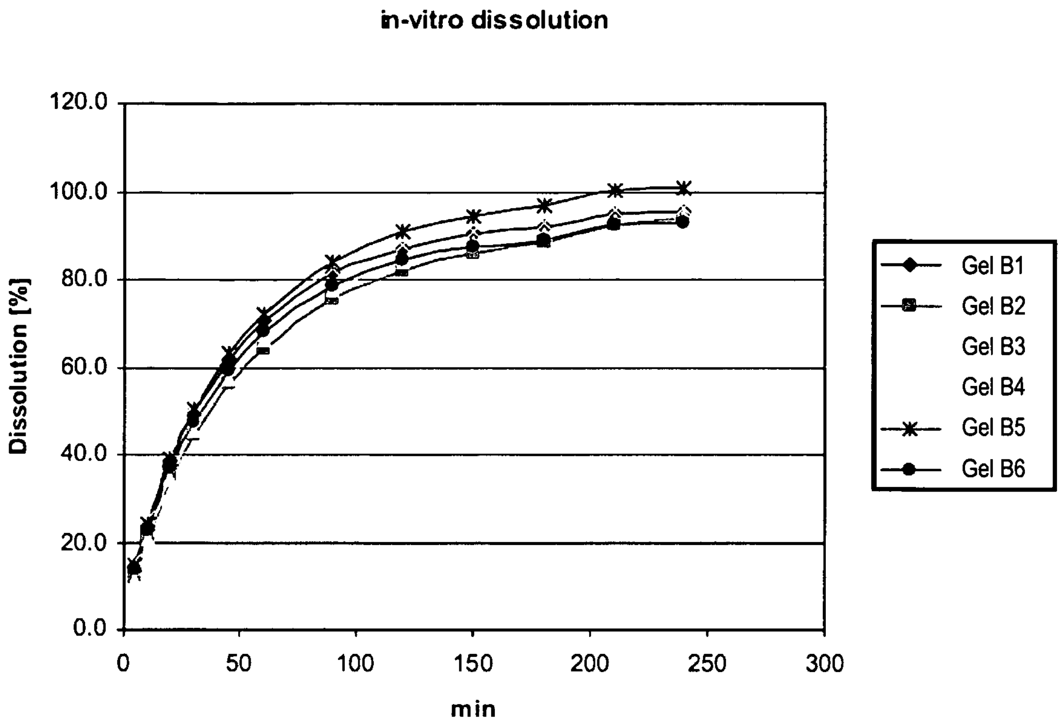


Figure 4: Composition B: Release (in-vitro-dissolution) of prilocaine hydrochloride, intermediate precision (n=6), paddle, 75 rpm

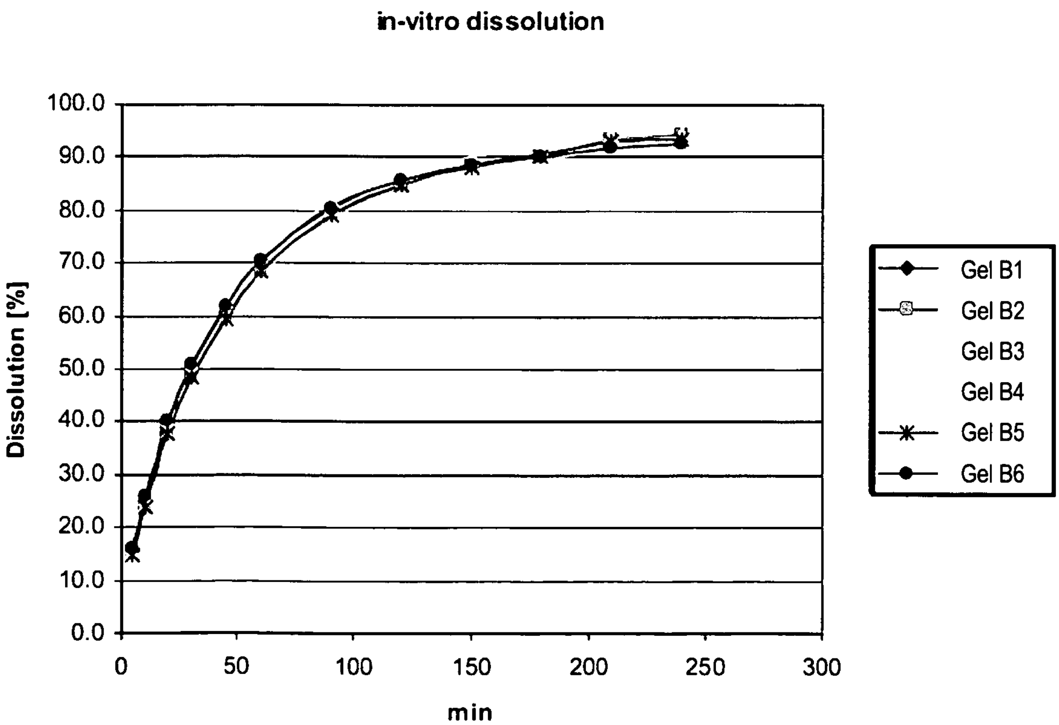

Figure 5: Composition C (Sample 1): Release (in-vitro-dissolution) of prilocaine hydrochloride, precision (n=6), paddle, 75 rpm

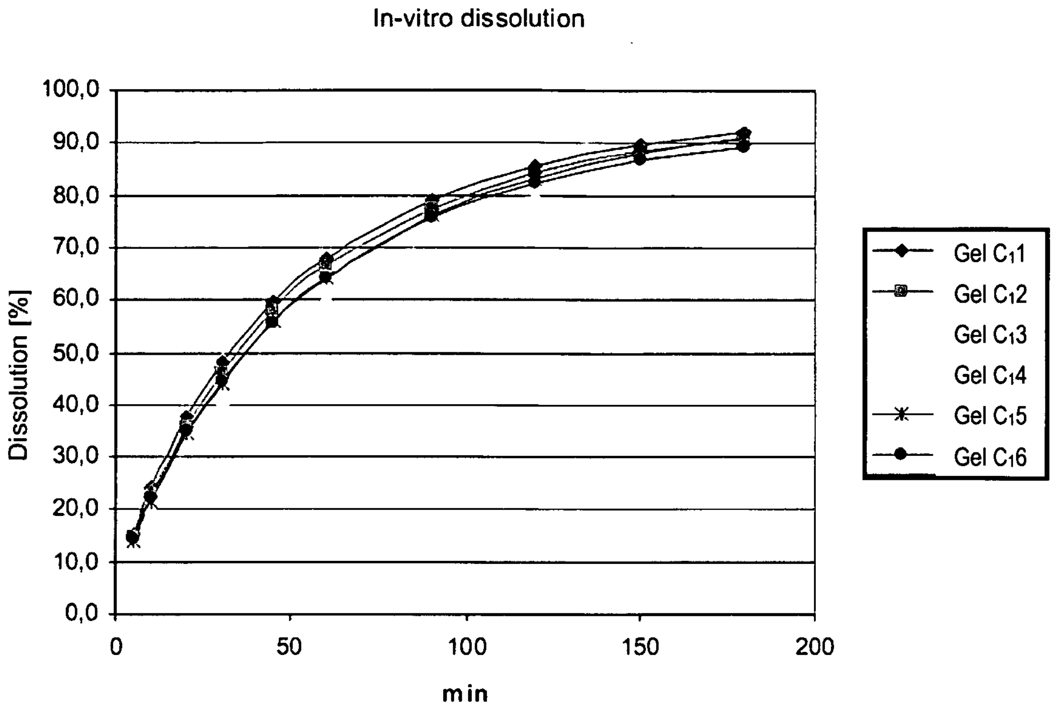


Figure 6: Composition C (Sample 1): Release (in-vitro-dissolution) of prilocaine hydrochloride, intermediate precision (n=6), paddle, 75 rpm

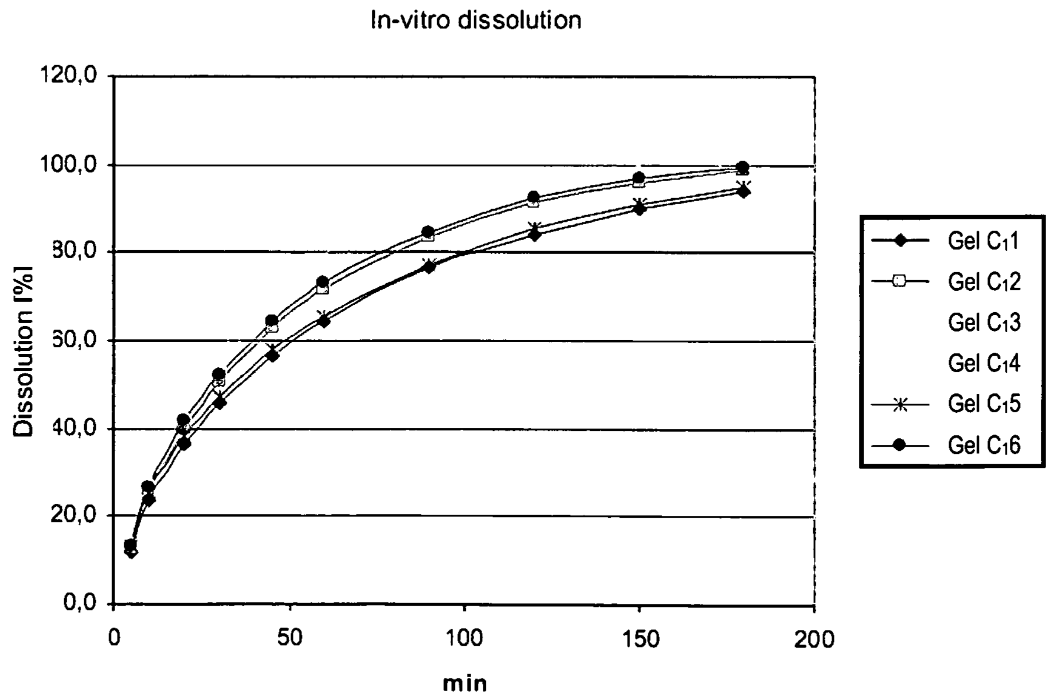

Figure 7: Composition C (Sample 2): Release (in-vitro-dissolution) of prilocaine hydrochloride, precision (n=6), paddle, 75 rpm

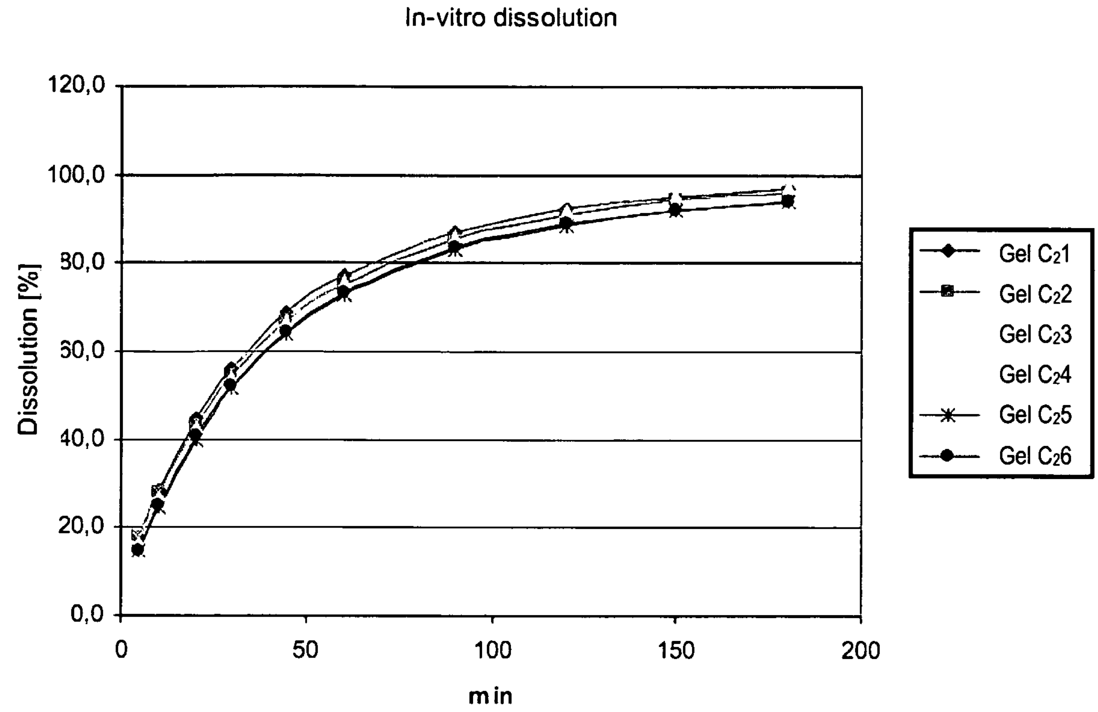


Figure 8: Composition X: Release (in-vitro-dissolution) of prilocaine hydrochloride, precision (n=6), paddle, 75 rpm

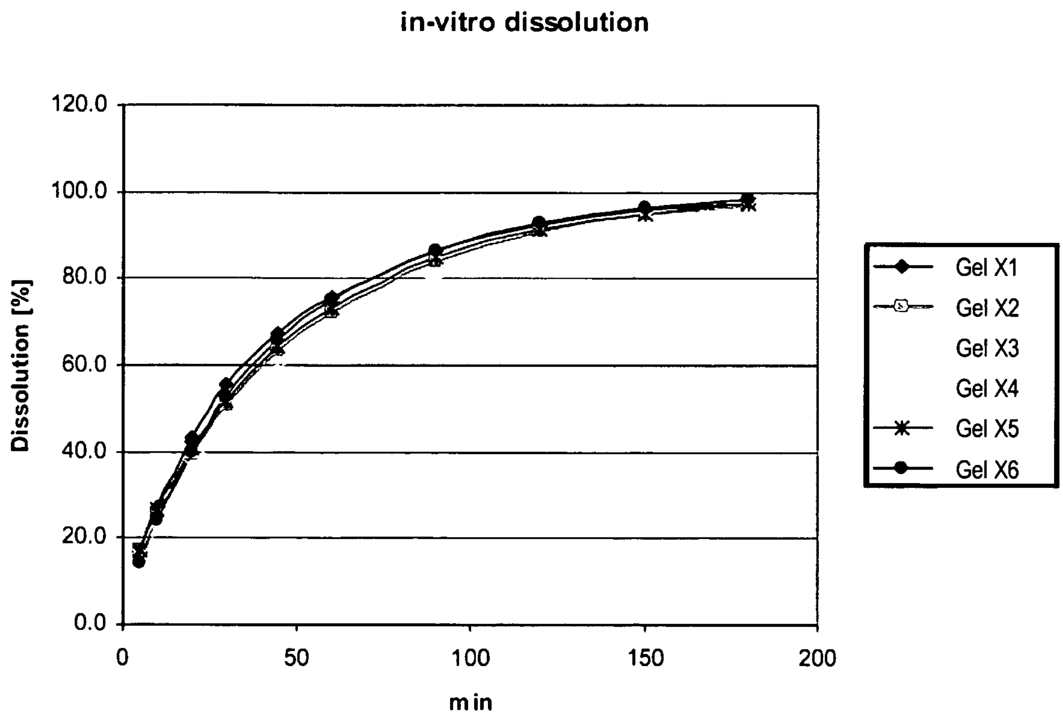

Figure 9: Composition X: Release (in-vitro-dissolution) of prilocaine hydrochloride, intermediate precision (n=6), paddle, 75 rpm

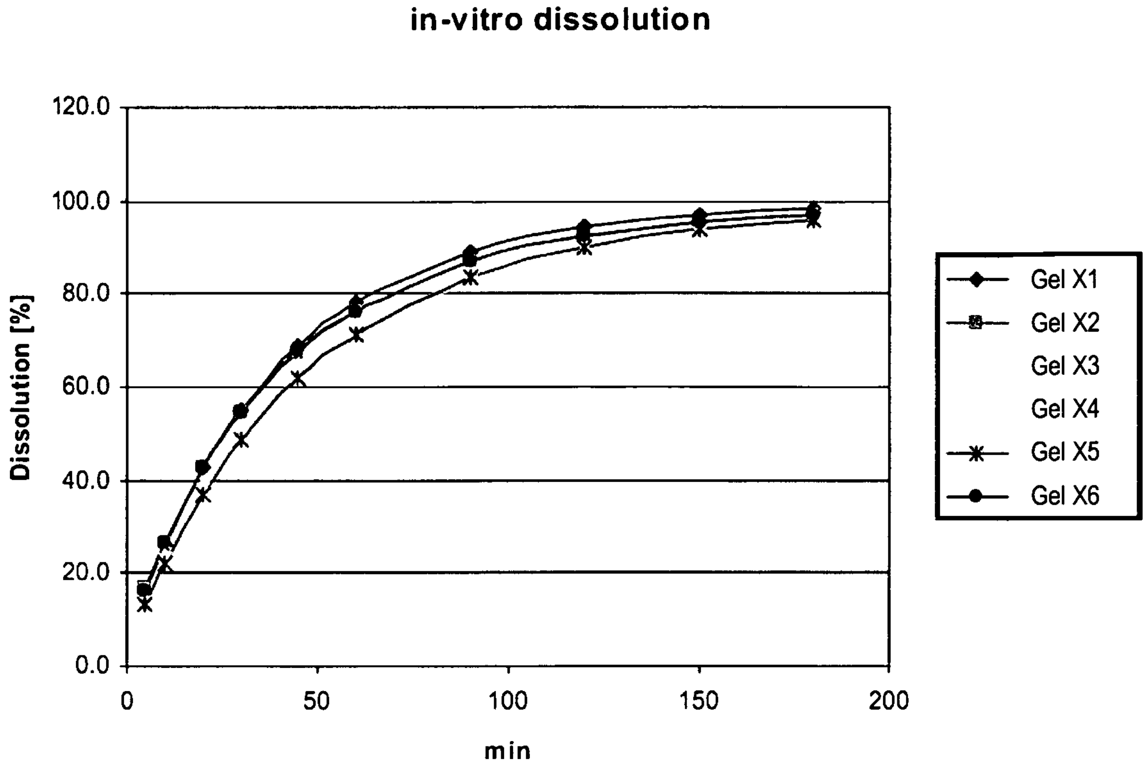

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2010015901 A1
- US 2010255068 A1
- US 5972326 A
- WO 0037047 A
- US 6224857 B
- US 20060122147 A
- WO 9822598 A


### Non-patent literature cited in the description


- *CHEMICAL ABSTRACTS,* 721-50-6
- *CHEMICAL ABSTRACTS,* 1786-81-8
- **UENO et al.** *Chem. Pharm. Bull.,* 1988, vol. 36, 4971-4975
- **WYATT.** *Anal. Chim. Acta,* 1993, vol. 272, 1-40
- Light Scattering University DAWN Course Manual. Wyatt Technologies. 1999
- DAWN EOS Manual. Wyatt Technology Corporation